# EUROPEAN PATENT SPECIFICATION

(11) **EP 0 894 140 B1**
(45) Date of publication and mention of the grant of the patent: **13.08.2008**
(21) Application number: 97906817.8
(22) Date of filing: 03.03.1997
(51) Int. Cl.: C12N 15/11, A61K 48/00, A61K 31/713

(54) **ALLELE SUPPRESSION**
ALLEL-ABBAU
SUPPRESSION D'ALLELES

(30) Priority: 01.03.1996 GB 9604449
(43) Date of publication of application: 03.02.1999
(73) Proprietor: Optigen Patents Limited, Dublin 2 (IE)
(72) Inventor: FARRAR, Gwenyth Jane, Monkstown D20, County Dublin (IE); HUMPHRIES, Peter, Cabinteely D15, County Dublin (IE); KENNA, Paul, Francis, Dublin 7 (IE)
(74) Representative: Main, Malcolm Charles
(86) International application number: PCT/GB1997/000574
(87) International publication number: WO 1997/032024

(56) References cited:
- WO-A-92/12262
- WO-A-94/11494
- WO-A-94/26887
- WO-A-95/03335
- WO-A-95/34573
- WO-A-97/11169
- LEUKEMIA, vol. 8, no. SUPPL. 01, April 1994, pages 152-155, XP000652853 ROBINSON-BENION C ET AL: "GENE TRANSPLANTATION: COMBINED ANTISENSE INHIBITION AND GENE REPLACEMENT STRATEGIES"
- NATURE, vol. 371, 13 October 1994, LONDON GB, pages 619-622, XP002033257 SULLENGER, B. & CECH, T.: "Ribozyme-mediated repair of defective mRNA by targeted trans-splicing" cited in the application

## Description

The present invention relates to a strategy for suppressing a gene. In particular the invention relates to suppression of mutated genes which give rise to a dominant or deleterious effect, either monogenically or polygenically.

Studies of degenerative hereditary ocular conditions, including Retinitis Pigmentosa (RP) and various macular dystrophies have resulted in a substantial elucidation of the molecular basis of these debilitating human retinal degenerations. Applying the approach of genetic linkage, x-linked RP (xlRP) genes have been localised to the short arm of the X chromosome (Ott et al. 1990) - subsequently the gene involved in one form of xlRP has been identified. Various genes involved in autosomal dominant forms of RP (adRP) have been localised. The first of these mapped on 3q close to the gene encoding the rod photoreceptor protein rhodopsin (McWilliam et al. 1989; Dryja et al. 1990). Similarly, an adRP gene was placed on 6p close to the gene encoding the photoreceptor protein peripherin (Farrar et al. 1991a,b; Kajiwara et al. 1991). Other adRP genes have been mapped to discrete chromosomal locations however the disease genes as yet remain uncharacterised. As in xlRP and adRP, various genes involved in autosomal recessive RP (arRP) have been localised and in some cases molecular defects characterised (Humphries et al. 1992; Farrar et al. 1993; Van Soest et al. 1994). Similarly a number of genes involved in macular dystrophies have been mapped (Mansergh et al. 1995). Genetic linkage, together with techniques for mutational screening of candidate genes, enabled identification of causative dominant mutations in the genes encoding rhodopsin and peripherin. Globally about 100 rhodopsin mutations have been found in patients with RP or congenital stationary night blindness. Similarly approximately 40 mutations have been characterised in the peripherin gene in patients with RP or macular dystrophies. Knowledge of the molecular aetiology of these retinopathies has stimulated the generation of animal models and the exploration of methods of therapeutic intervention (Farrar et al. 1995; Humphries et al. 1997).

Similar to RP, osteogenesis imperfecta (OI) is an autosomal dominantly inherited human disorder whose molecular pathogenesis is extremely genetically heterogeneous. OI is often referred to as Tbrittle bone diseaseU although additional symptoms including hearing loss, growth deficiency, bruising, loose joints, blue sclerae and dentinogenesis imperfeca are frequently observed (McKusick, 1972). Mutations in the genes encoding the two type I collagen chains (collagen 1A1 or 1A2) comprising the type I collagen heterodimer have been implicated in OI. Indeed hundreds of dominantly acting mutations have been identified in OI patients in these two genes, many of which are single point mutations, although a number of insertion and deletion mutations have been found (Willing et al. 1993; Zhuang et al. 1996). Similarly mutations in these genes have also been implicatd in to Ehlers -Danlos and Marfan syndromes (Dalgleish et al. 1986; Phillips et al. 1990; DUAlessio et al. 1991; Vasan NS et al. 1991).

Generally, gene therapies utilising viral and non-viral delivery systems have been used to treat inherited disorders, cancers and infectious diseases. However, many studies have focused on recessively inherited disorders, the rationale being that introduction and expression of the wild type gene may be sufficient to prevent/ameliorate the disease phenotype. In contrast gene therapy for dominant disorders will require suppression of the dominant disease allele. Notably many of the characterised mutations causing inherited diseases such as RP or OI are inherited in an autosomal dominant fashion. Indeed there are over 1,000 autosomal dominantly inherited disorders in man. In addition there are many polygenic disorders due to co-inheritance of a number of genetic components which together give rise to the disease state. Effective gene therapies for dominant or polygenic diseases may be targeted to the primary defect and in this case may require suppression of the disease allele while in many cases still maintaining the function of the normal allele. Alternatively suppression therapies may be targeted to secondary effects associated with the disease pathology: one example is programmed cell death (apoptosis) which has been observed in many inherited disorders.

Strategies to differentiate between normal and disease alleles and to selectively switch off the disease allele using suppression effectors interalia antisense DNA/RNA, PNAs, ribozymes or triple helix DNA, targeted towards the disease mutation may be difficult in many cases - frequently disease and normal alleles differ by only a single nucleotide. A further difficulty inhibiting development of gene therapies is the heterogeneous nature of some dominant disorders - many different mutations in the same gene give rise to a similar disease phenotype. Development of specific gene therapies for each of these may be prohibitive in terms of cost. To circumvent difficulties associated with specifically targeting the disease mutation and with the genetic heterogeneity present in inherited disorders, a novel strategy for gene suppression exploiting polymorphism, thereby allowing some flexibility in choice of target sequence for suppression and providing a means of gene suppression which is independent of the disease mutation, is described in the invention.

Suppression effectors have been used previously to achieve specific suppression of gene expression. Antisense DNA and RNA has been used to inhibit gene expression in many instances. Modifications, such as phosphorothioates, have been made to oligonucleotides to increase resistance to nuclease degradation, binding affinity and uptake (Cazenave et al. 1989; Sun et al. 1989; McKay et al. 1996; Wei et al. 1996). In some instances, using antisense and ribozyme suppression strategies has led to reversal of a tumour phenotype by reducing expression of a gene product or by cleaving a mutant transcript at the site of the mutation (Carter and Lemoine 1993; Lange et al. 1993; Valera et al. 1994; Dosaka-Akita et al. 1995; Feng et al. 1995; Quattrone et al. 1995; Ohta et al. 1996). For example, neoplastic reversion was obtained using a ribozyme targeted to a H-ras mutation in bladder carcinoma cells (Feng et al. 1995). Ribozymes have also been proposed as a means of both inhibiting gene expression of a mutant gene and of correcting the mutant by targeted trans-splicing (Sullenger and Cech 1994; Jones et al. 1996). Ribozymes can be designed to elicit autocatalytic cleavage of RNA targets, however, the inhibitory effect of some ribozymes may be due in part to an antisense effect due to the antisense sequences flanking the catalytic core which specify the target site (Ellis and Rodgers 1993; Jankowsky and Schwenzer 1996). Ribozyme activity may be augmented by the use of, for example, non-specific nucleic acid binding proteins or facilitator oligonucleotides (Herschlag et al. 1994; Jankowsky and Schwenzer 1996). Multitarget ribozymes (connected or shotgun) have been suggested as a means of improving efficiency of ribozymes for gene suppression (Ohkawa et al. 1993). Triple helix approaches have also been investigated for sequence specific gene suppression - triplex forming oligonucleotides have been found in some cases to bind in a sequence specific manner (Postel et al. 1991; Duval-Valentin et al. 1992; Hardenbol and Van Dyke 1996; Porumb et al. 1996). Similarly peptide nucleic acids have been shown to inhibit gene expression (Hanvey et al. 1992; Knudson and Nielsen 1996; Taylor et al. 1997). Minor groove binding polyamides can bind in a sequence specific manner to DNA targets and hence may represent useful small molecules for future suppression at the DNA level (Trauger et al. 1996). In addition, suppression has been obtained by interference at the protein level using dominant negative mutant peptides and antibodies (Herskowitz 1987; Rimsky et al. 1989; Wright et al. 1989). In some cases suppression strategies have lead to a reduction in RNA levels without a concomitant reduction in proteins, whereas in others, reductions in RNA have been mirrored by reductions in protein.

There is now an armament with which to obtain gene suppression. This, in conjunction with a better understanding of the molecular etiology of disease, results in an ever increasing number of disease targets for therapies based on suppression. In many cases, complete suppression of gene expression has been difficult to achieve. Possibly a combined approach using a number of suppression effectors may aid in this. For some disorders it may be necessary to block expression of a disease allele completely to prevent disease symptoms whereas for others low levels of mutant protein may be tolerated. In parallel with an increased knowledge of the molecular defects causing disease has been the realisation that many disorders are genetically heterogeneous. Examples in which multiple genes and/or multiple mutations within a gene can give rise to a similar disease phenotype include osteogenesis imperfecta, familial hypercholesteraemia, retinitis pigmentosa, and many others. In addition to the genetic heterogeneity inherent in inherited disorders there has been significant elucidation of the polymorphic nature of the human genome and indeed the genome of other species. Polymorphisms inter alia simple sequence repeats, insertions, deletions or single nucleotide changes (either silent changes or changes resulting in amino acid substitutions) have been observed in many human genes. As the human genome sequencing project proceeds levels of polymorphism in the genome are being more accurately defined and increasing numbers of intragenic polymorphisms are becoming available. Polymorphisms have been found in coding and non-coding sequences of most genes explored. Coding sequence is under greater evolutionary constraint than non-coding sequence limiting the degree of polymorphism and the nature of that polymorphism - one would predict that fewer polymorphisms involving significant changes, for example, multiple nucleotides will be found in coding sequence. However it is likely that such polymorphisms will be useful in optimising strategies for gene suppression of individual alleles, for example, a 38bp insertion found in collgen 1A2 may be useful in optimising suppression of alleles of this gene carrying tis insertion (Dalgleish et al. 1986). The utility of polymorphism to discriminate between alleles where one allele also carries a mutation which is independent of the polymorphism and which causes abnormal or deleterious cell functioning or cell death has been exploited in the invention.

Polymorphism has in the prior art been proposed as a method to suppress one allele of a gene(s) whose product(s) is vital to cell viability - this has been proposed particularly in relation to treatment of tumours where one allele is absent in tumour cells and therefore suppression of the second allele which is vital for cell viability may result in induction of tumour cell death while non-tumourous diploid cells should in principle remain viable as they should still maintain one functioning wild type allele even after the suppression therapy has been applied (D.E. Housman PCT/US94/08473).

The invention aims to address shortcomings of the prior art by providing a novel approach to the design of suppression effectors directed to target alleles of a gene carrying a deleterious mutation. Suppression of every mutation giving rise to a disease phenotype may be costly and problematic. Disease mutations are often single nucleotide changes. As a result differentiating between the disease and normal alleles may be difficult. Some suppression effectors require specific sequence targets, for example, hammerhead ribozymes cleave at NUX sites and hence may not be able to target many mutations. Notably, the wide spectrum of mutations observed in many diseases adds additional complexity to the development of therapeutic strategies for such disorders - some mutations may occur only once in a single patient. A further problem associated with suppression is the high level of homology present in coding sequences between members of some gene families. This can limit the range of target sites for suppression which will enable specific suppression of a single member of such a gene family - polymorphic sites within a gene may be the most appropriate sequences to enable specific targeting.

The present invention circumvents shortcomings in the prior art utilising polymorphism. In the invention suppression effectors are designed specifically to target polymorphic sites in regions of genes or gene products where one allele of the gene contains a mutation with a deleterious effect which is not causally associated with the polymorphism. This provides more flexibility in choice of target sequence for suppression in contrast to suppression strategies directed towards single disease mutations as many genes have multiple polymorphic target sites.

According to the present invention there is provided an in vitro method for suppressing expression of one allele of an endogenous gene with a deleterious mutation, wherein said method comprises (i) providing suppression effectors such as antisense nucleic acids able to bind to polymorphisms in coding regions within a gene such that one allele of a gene is exclusively or preferentially suppressed, and (ii) providing replacement nucleic acids encoding a replacement gene which is a different naturally occurring allele, wherein the replacement nucleic acid has a sequence which allows it to be expressed and escape complete suppression.

Generally the term suppression effectors means the nucleic acids, peptide nucleic acids (PNAs), peptides, antibodies or modified forms of these used to silence or reduce gene expression in a sequence specific manner.

Suppression effectors, such as antisense nucleic acids can be DNA or RNA, and are directed to coding regions within said gene. Binding of the suppression effector(s) prevents or lowers functional expression of one allele of the endogenous gene carrying a deleterious mutation by targeting polymorphism(s) in coding regions within or adjacent to the gene.

Generally the term "functional expression" means the expression of a gene product able to function in a manner equivalent to or better than a wild type product. In the case of a mutant gene or predisposing gene "functional expression" means the expression of a gene product whose presence gives rise to a deleterious effect or predisposes to a deleterious effect. By deleterious effect is meant giving rise to or predisposing to disease pathology or altering the effect(s) and/or efficiency of an administered compound.

In a particular embodiment of the invention the strategy further employs ribozymes which can be designed to elicit cleavage of target R7NAs. The strategy further employs nucleotides which form triple helix DNA. Nucleic acids for antisense, ribozymes and triple helix may be modified to increase stability, binding efficiencies and uptake (see prior art). Nucleic acids can be incorporated into a vector. Vectors include naked DNA, DNA plasmid vectors, RNA or DNA virus vectors, lipids, polymers or other derivatives and compounds to aid gene delivery and expression.

The invention further provides the use of antisense nucleotides, ribozymes, PNAs, triple helix nucleotides or other suppression effectors alone or in a vector or vectors, wherein the nucleic acids are able to bind specifically or partially specifically to one allele of a gene to prevent or reduce the functional expression thereof, wherein said allele contains a mutation having a deleterious effect wherein the suppression effectors bind a polymorphism in coding regions within said gene, wherein said polymorphism is not causally associated with said deleterious effect, and replacement nucleic acids which are a different naturally occurring allele, wherein the replacement nucleic acid has a sequence which allows it to be expressed and escape complete suppression, in the preparation of a medicament for the treatment of an autosomal dominant or polygenic disease.

Described herein is a strategy for suppressing specifically or partially specifically one allele of an endogenous gene with a deleterious mutation(s) and if required introducing a replacement gene, said strategy comprising the steps of:
1. providing nucleic acids able to bind to at least one allele of a gene to be suppressed and
2. providing genomic DNA or cDNA (complete or partial) encoding a replacement gene which is a different allele (either a naturally occurring or artificially derived allelic variant) than the allele targeted for suppression, wherein the nucleic acids are unable to bind to equivalent regions in the genomic DNA or cDNA to prevent expression of the replacement gene. The replacement nucleic acids will not be recognised by suppression nucleic acids or will be recognised less effectively than the allele targeted by suppression nucleic acids.

In a particular embodiment of the invention there is provided a strategy for gene suppression targeted to a particular characteristic associated with one allele of the gene to be suppressed. Suppression will be specific or partially specific to one allele, for example, to the allele carrying a deleterious mutation. The invention further employs use of replacement nucleic acids such that replacement nucleic acids will not be recognised (or will be recognised less effectively) by suppression nucleic acids which are targeted specifically or partially specifically to one allele of the gene to be suppressed.

Replacement nucleic acids may be provided such that replacement nucleic acids will not be recognised by naturally occurring suppressors found to inhibit or reduce gene expression in one or more individuals, animals or plants. The invention uses of replacement nucleic acids which have altered sequences around polymorphic site(s) targeted by suppressors of the gene such that suppression by naturally occurring suppressors is completely or partially prevented.

Replacement nucleic acids representing a different allele from the allele targeted by suppression effectors and which provide a normal gene product which is equivalent to or improved compared with the naturally occurring endogenous gene product may be need in the invention.

A strategy may be used to suppress one allele of a gene using polymorphism where that allele or the product of that allele interferes with the action of an administered compound.

The invention further provides the use of a vector or vectors containing suppression effectors in the form of nucleic acids, said nucleic acids being directed towards polymorphic sites in coding regions within the target gene and vector(s) containing genomic DNA or cDNA encoding a replacement gene sequence to which nucleic acids for suppression are unable to bind (or bind less efficiently), in the preparation of a combined medicament for the treatment of an autosomal dominant or polygenic disease. Nucleic acids for suppression or replacement gene nucleic acids may be provided in the same vector or in separate vectors. Nucleic acids for suppression or replacement gene nucleic acids may be provided as a combination of nucleic acids alone or in vectors.

Described herein is a method of treatment for a disease caused by an endogenous mutant gene, said method comprising sequential or concomitant introduction of (a) nucleic acids to one allele of a gene to be suppressed; suppression being targeted to polymorphism(s) in coding regions within said gene, (b) replacement nucleic acids with sequences which allow it to be expressed.

The nucleic acid for gene suppression can be administered before, after or at the same time as the replacement gene is administered.

The invention further provides a kit for use in the treatment of a disease caused by a deleterious mutation in a gene, the kit comprising nucleic acids for suppression able to bind one allelic variant of the gene to be suppressed wherein the suppression effectors bind a polymorphism in coding regions within said gene to be suppressed, wherein said polymorphism is not causally associated with said deleterious effect and a replacement nucleic acid which is a different naturally occurring allele to replace the mutant gene having sequence which allows it to be expressed and completely or partially escape suppression.

Nucleotides can be administered as naked DNA or RNA. Nucleotides can be delivered in vectors. Naked nucleic acids or nucleic acids in vectors can be delivered with lipids or other derivatives which aid gene delivery. Nucleotides may be modified to render them more stable, for example, resistant to cellular nucleases while still supporting RNaseH mediated degradation of RNA or with increased binding efficiencies (see prior art). Antibodies or peptides can be generated to target the protein product from one allele of the gene to be suppressed.

The invention relates to a strategy for suppressing a gene or disease allele using methods which do not target the disease allele specifically but instead targets a polymorphism in coding regions within said gene. A particular embodiment of the invention is the use of polymorphism within a gene to direct suppression strategies to the disease allele while still allowing continued expression of the normal allele. The strategy circumvents the need for a specific therapy for every mutation within a given gene. In addition the invention allows greater flexibility in choice of target sequence for suppression of a disease allele.

Desribed also is a medicament or medicaments for use in suppressing a deleterious allele which is present in a genome of one or more individuals or animals.

Generally the present invention will be useful where the gene, which is naturally present in the genome of a patient, contributes to a disease state. Generally, one allele of the gene in question will be mutated, that is, will possess alterations in its nucleotide sequence that affects the function or level of the gene product.
For example, the alteration may result in an altered protein product from the wild type gene or altered control of transcription and processing. Inheritance or somatic acquisition of such a mutation can give rise to a disease phenotype or can predispose an individual to a disease phenotype. However the gene of interest could also be of wild type phenotype, but contribute to a disease state in another way such that the suppression of the gene would alleviate or improve the disease state or improve the effectiveness of an administered therapeutic compound.

Generally, suppression effectors such as nucleic acids - antisense or sense, ribozymes, peptide nucleic acids (PNAs), triple helix forming oligonucleotides, peptides and /or antibodies directed to polymorphisms in a gene, in transcripts or in protein, can be employed in the invention to achieve gene suppression.

Notably, the invention has the advantage that the same suppression strategy when directed to polymorphisms could be used to suppress, in principle, many mutations in a gene. This is particularly relevant when large numbers of mutations within a single gene cause disease pathology. The proportion of disease mutations which can be suppressed using a polymorphism will depend in part on the frequency of the polymorphism chosen for suppression in the population. Multiple polymorphims may be chosen to increase the proportion of individuals that can be targeted. Suppression using one allele of a polymorphism enables when necessary the introduction of a replacement gene with a different allele of the polymorphism such that the replacement gene escapes suppression completely or partially as does the normal endogenous allele. The replacement gene provides (when necessary) additional expression of the normal protein product when required to ameliorate pathology associated with reduced levels of wild type protein. The same replacement gene could in principle be used in conjunction with the suppression of many different disease mutations within a given gene. Target polymorphisms may be found either in coding or non-coding sequence or in regions 5' or 3' of the gene. For example, intronic polymorphisms could be used for suppression. The use of polymorphic targets for suppression in 5' and 3' non-coding sequence holds the advantage that such sequences are present in both precursor and mature RNAs, thereby enabling suppressors to target all forms of RNA. In contrast, intronic sequences are spliced out of mature transcripts. Similarly polymorphims found in coding sequence would be present in precursor and mature transcripts again enabling suppressors to target all forms of RNA. Polymorphisms in coding sequence may be silent and have no effect on subsequent protein amino acid content or may result in an amino acid substitution but not lead to a disease pathology. In the latter case, such polymorphisms may enable targeting of one allele specifically at the protein level by directing, for example, antibodies, uniquely to one form of the protein.

In summary the invention can involve gene suppression of one allele targeting polymorphism(s) in coding regions within the gene and when necessary gene replacement such that the replacement gene cannot be suppressed, that is, it represents a different allelic form from that targeted for suppression. The same suppression and replacement steps can be used for many disease mutations in a given gene - the invention enables the same approach to be used to suppress a wide range of mutations within the same gene. Suppression and replacement can be undertaken in conjunction with each other or separately.

The strategy described herein has applications for alleviating autosomal dominant diseases. Complete silencing of a disease allele may be difficult to achieve using antisense, PNA, ribozyme and triple helix approaches or any combination of these. However small quantities of mutant product may be tolerated in some autosomal dominant disorders. In others a significant reduction in the proportion of mutant to normal product may result in an amelioration of disease symptoms. Hence this strategy may be applied to any autosomal dominantly or polygenically inherited disease in man where the molecular basis of the disease has been established. This strategy will enable the same therapy to be used to treat a range of different disease mutations within the same gene. The development of strategies will be important to future therapies for autosomal dominant and polygenic diseases, the key to a general strategy being that it circumvents the need for a specific therapy for every mutation causing or predisposing to a disease. This is particularly relevant in some disorders, for example, rhodopsin linked autosomal dominant RP, in which to date about one hundred different mutations in the rhodopsin gene have been observed in adRP patients. Likewise hundreds of mutations have been identified in the human type I Collagen 1A1 and 1A2 genes in autosomal dominant osteogenesis imperfecta. Costs of developing therapies for each mutation are prohibitive at present. Inventions such as this one using a general approach for therapy will be required. General approaches may be targeted to the primary defect as is the case with this invention or to secondary effects such as apoptosis.

This invention may be applied in gene therapy approaches for biologically important polygenic disorders affecting large proportions of the world's populations such as age related macular degeneration, glaucoma, manic depression, cancers having a familial component and indeed many others. Polygenic diseases require inheritance of more than one mutation (component) to give rise to the disease state. Notably an amelioration in disease symptoms may require reduction in the presence of only one of these components, that is, suppression of one genotype which, together with others leads to the disease phenotype, may be sufficient to prevent or ameliorate symptoms of the disease. In some cases suppression of more than one component may be required to improve disease symptoms. This invention may be applied in possible future interventive therapies for common polygenic diseases to suppress a particular genotype(s) using polymorphisms and thereby suppress the disease phenotype.

### Examples

The present invention is exemplified herein using three genes: human rhodopsin and human Collagen 1A1 and 1A2. The first of these genes is retinal specific. In contrast, Collagen 1A1 and 1A2 are expressed in a range of tissues including skin and bone. While these three genes have been used as examples there is no reason why the invention could not be deployed in the suppression of individual allelic variants of many other genes in which mutations cause or predispose to a deleterious effect. Many examples of mutant genes which give rise to disease phenotypes are available from the prior art. Similarly, many polymorphisms have been identified in genes in which disease causing mutations have been observed - these genes all represent targets for the invention. The present invention is exemplified using hammerhead ribozymes with antisense arms to elicit RNA cleavage. There is no reason why other suppression effectors directed towards individual polymorphic variants of genes or gene products could not be used to achieve gene suppression. Many examples from the prior art detailing use of suppression effectors inter alia antisense RNA/DNA, triple helix, PNAs and peptides to achieve suppression of gene expression are reported (see prior art). The present invention is exemplified using hammerhead ribozymes with antisense arms to elicit sequence specific cleavage of transcripts transcribed from one vector and containing one allele of a polymorphism and non-cleavage of transcripts containing a different allelic variant of a polymorphism. Uncleavable alleles could be used in a replacement genes if required to restore levels of wild type protein thereby preventing pathology due to haplo-insufficiency. The present invention is exemplified using suppression effectors directed to target single allelic variants of human rhodopsin and human Collagen 1A1 and 1A2 targeting polymorphic sites in coding or 3' untranslated regions of the genes. There is no reason why polymorphisms in other transcribed but untranslated regions of genes or in introns or in regions involved in the control of gene expression such as promoter regions or in regions adjacent to the gene or any combination of these could not be used to achieve gene suppression. Suppression targeted to any polymorphism within or close to a gene may allow selective suppression of one allele of the gene carrying a deleterious mutation while maintaining expression of the other allele. Multiple suppression effectors for example shotgun ribozymes could be used to optimise efficiency of suppression when necessary. Additionally when required expression of a replacement gene with an allelic variant different to that to which suppression effector(s) are targeted may be used to restore levels of wild type gene product.

The examples are illustrated with reference to the accompanying drawings wherein
Diagram 1 shows pBR322 cut with MspI for use as a DNA ladder.
Figure 1A illustrates human rhodopsin cDNA expressed from the T7 promoter to the BstEII site in the coding sequence.
Figure 1B illustrates unadapted human rhodopsin cDNA expressed from the T7 promoter to the FspI site in the coding sequence.
Figure 2A illustrates unadapted and adapted human rhodopsin cDNAs expressed from the T7 promoter to the AcyI after the coding sequence and the BstEII site in the coding sequence respectively.
Figure 2B illustrates the adapted human rhodopsin cDNA was expressed from the T7 promoter to the BstEII site in the coding sequence.
Figure 2C illustrates unadapted and adapted human rhodopsin cDNAs expressed from the T7 promoter to the AcyI after the coding sequence and the BstEII site in the coding sequence respectively.
Figure 3 illustrates mutant (Pro23Leu) human rhodopsin cDNA expressed from the T7 promoter to the BstEII in the coding sequence.
Figure 4 illustrates mutant (Pro23Leu) human rhodopsin cDNA expressed from the T7 promoter to the BstEII in the coding sequence.
Figure 5A illustrates human collagen 1A1 cDNA clones containing the T allele of the polymorphism at 3210 expressed from the T7 promoter to the XbaI site in the vector.
Figure 5B illustrates human collagen 1A1 cDNA clones containing the C allele of the polymorphism at 3210 expressed from the T7 promoter to the XbaI site in the vector.
Figure 6 illustrates human collagen 1A1 cDNA clones containing the T allele of the polymorphism at 3210 expressed from the T7 promoter to the XbaI site in the vector.
Figure 7 illustrates human collagen 1A1 cDNA clones containing the C allele of the polymorphism at 3210 expressed from the T7 promoter to the XbaI site in the vector.
Figure 8A illustrates human collagen 1A2 cDNA clones containing the A and T alleles of the polymorphism at position 907 expressed from the T7 promoter to the MvnI and XbaI sites in the insert and vector respectively.
Figure 8B illustrates human collagen 1A2 cDNA (A) + (B) clones containing the A and T alleles of the polymorphism at 907 expressed from the T7 promoter to the MvnI and XbaI sites in the insert and vector respectively.
Figure 9 illustrates A: The human collagen 1A2 cDNA (A) and (B) clones containing the A and G alleles of the polymorphism at position 902 expressed from the T7 promoter to the MvnI and XbaI sites in the insert and vector respectively.

### Sequences

### Sequence 1

The human rhodopsin cDNA in pCDNA3.

### Sequence 2

The human rhodopsin cDNA in pCDNA3 with a base change at a silent site (477).

### Sequence 3

Mutant (Pro23Leu) human rhodopsin cDNA in pCDNA3.

### Sequence 4

Rz10 cloned into pCDNA3. Note there is a one base mismatch in one antisense arm of Rz10.

### Sequence 5

Rz20 cloned into pCDNA3

### Sequence 6

Collagen 1A1 (A) sequence containing the T polymorphism at 3210

### Sequence 7

Collagen 1A1 (B) sequence containing the C polymorphism at 3210

### Sequence 8

RzPolCol1A1 cloned into pCDNA3

### Sequence 9

Collagen 1A2 (A) sequence containing the G and T polymorphisms at positions 902 and 907 respectively.

### Sequence 10

Collagen 1A2 (B) sequence containing the A and A polymorphisms at positions 902 and 907 respectively.

### Sequence 11

Rz902 cloned into pCDNA3

### Sequence 12

Rz902 cloned into pCDNA3

### MATERIALS and METHODS

### Cloning vectors

cDNA templates and ribozymes DNA fragments were cloned into commercial expression vectors (pCDNA3, pZeoSV or pBluescript) which enable expression in a test tube from T7, T3 or SP6 promoters or expression in cells from CMV or SV40 promoters. Inserts were placed into the multiple cloning site (MCS) of these vectors typically at or near the terminal ends of the MCS to delete most of the MCS and thereby prevent any possible problems with efficiency of expression subsequent to cloning.

### Sequencing protocols

Clones containing template cDNAs and ribozymes were sequenced by ABI automated sequencing machinery using standard protocols.

### Expression of RNAs

RNA was obtained from clones in vitro using a commercially available Ribomax expression system (Promega) and standard protocols. RNA purifications were undertaken using the Bio-101 RNA purification kit or a solution of 0.3M sodium acetate and 0.2% SDS after running on polyacrylamide gels. Cleavage reactions were performed using standard protocols with varying MgCl2 concentrations (0-15mM) at 37 °C typically for 3 hours. Time points were performed at the predetermined optimal MgC12 concentrations for up to 5 hours. Radioactively labelled RNA products were obtained by incorporating a-P32 rUTP (Amersham) in the expression reactions (Gaughan et al. 1995). Labelled RNA products were run on polyacrylamide gels before cleavage reactions were undertaken for the purposes of RNA purification and subsequent to cleavage reactions to establish if RNA cleavage had been achieved. Cleavage reactions were undertaken with 5mM Tris-HCl Ph8.0 and varying concentrations of MgCl2 at 37°C.

### RNA secondary structures

Predictions of the secondary structures of human rhodopsin and human collagens 1A1 and 1A2 mRNAs was obtained using the RNAPlotFold program. Ribozymes and antisense were designed to target areas of the RNA that were predicted to be accessible to suppression effectors. The integrity of open loop structures was evaluated from the 10 most probable RNA structures. Additionally RNA structures for truncated RNA products were generated and the integrity of open loops between full length and truncated RNAs compared. RNA structures for 6 mutant rhodopsin transcripts were generated and the "robust nature" of open loop structures targeted by ribozymes compared between mutant transcripts (Table 2).

### TEMPLATES and RIBOZYMES

### Human Rhodopsin

### Template cDNA

The human rhodopsin cDNA was cloned into the HindIII and EcoRI sites of the MCS of pCDNA3 in a 5' to 3' orientation allowing subsequent expression of RNA from the T7 or CMV promoters in the vector. The full length 5'UTR sequence was inserted into this clone using primer driven PCR mutagenesis and a HindIII (in pCDNA3) to BstEII (in the coding sequence of the human rhodopsin cDNA) DNA fragment (Sequence 1)

Hybrid cDNAs with altered sequence resulting in an "artificial" polymorphism. The human rhodopsin hybrid cDNA with a single base alteration, a C-->G change (at position 477) was introduced into human rhodopsin cDNA using a HindIII to BstEII PCR cassette by primer directed PCR mutagenesis. This sequence change occurs at a silent position - it does not give rise to an amino acid substitution - however it eliminates the ribozyme cleavage site (GUX --> GUG). The hybrid rhodopsin was cloned into pCDNA3 in a 5' to 3' orientation allowing subsequent expression of RNA from the T7 or CMV promoters in the vector (Sequence 2).

Rhodopsin cDNA carrying a Pro123Leu adRP mutation A human rhodopsin adRP mutation, a single base alteration, a C-->T change (at codon 23) was introduced into human rhodopsin cDNA using a HindIII to BstEII PCR cassette by primer directed PCR mutagenesis. This sequence change results in the substitution of a Proline for a Serine residue. Additionally the nucleotide change creates a ribozyme cleavage site (CCC-->CTC). The mutated rhodopsin was cloned into the HindIII and EcoRI sites of pCDNA3 in a 5' to 3' orientation allowing subsequent expression of RNA from the T7 or CMV promoters in the vector (Sequence 3).

### Ribozyme constructs

A hammerhead ribozyme (termed Rz10) designed to target a large robust open loop structure in the RNA from the coding regions of the gene was cloned subsequent to synthesis and annealing into the HindIII and XbaI sites of pCDNA3 again allowing expression of RNA from the T7 or CMV promoters in the vector (Sequence 4). The target site was GUC (the GUX rule) at position 475-477 of the human rhodopsin sequence. A hammerhead ribozyme (termed Rz20) designed to target an open loop structure in RNA from the coding region of a mutant rhodopsin gene with a Pro23Leu mutation was cloned subsequent to synthesis and annealing into the HindIII and XbaI sites of pCDNA3 again allowing expression of RNA from the T7 or CMV promoters in the vector (Sequence 5). The target site was CTC (the NUX rule) at codon 23 of the human rhodopsin sequence (Accession number: K02281). Antisense flanks are underlined.
Rz 10: GGACGGTCTGATGAGTCCGTGAGGACGAAACGTAGAG
Rz20: TACTCGAACTGATGAGTCCGTGAGGACGAAAGGCTGC

### Human Type I Collagen - Col1A1

### Alleles A and B of Collagen 1A1

A section of the human collagen 1A1 cDNA was cloned from genomic DNA from 11 unrelated individuals into the HindIII and XbaI sites of pCDNA3. The clones were in a 5' to 3' orientation allowing subsequent expression of RNA from the T7 or CMV promoters in the vector (Sequences 6 + 7). The clones contain the Collagen 1A1 sequence from position 2977 to 3347 (Accession number: K01228). Clones containing allele A and B of a naturally occurring polymorphism in the 3'UTR (Westerhausen et al. 1990) and representing a T and a C nucleotide respectively at position 3210 were identified by sequence analysis.

### Ribozyme constructs

A hammerhead ribozyme (termed RzPolCol1A1) designed to target a large robust open loop structure (as determined from the ten most probable 2-D structures) in the RNA from the 3' UTR of the gene was cloned into the Hind III and XbaI sites of pCDNA3 again allowing subsequent expression of RNA from the T7 or CMV promoters in the vector (Sequence 8). The ribozyme target site was a GUX site at position 3209-3211 of the human Collagen 1A1 sequence (Accession number: K01228). Antisense flanks are underlined.
RzPolCol1A1: TGGCTTTTCTGATGAGTCCGTGAGGACGAAAGGGGGT

### Human Type I Collagen - COL1A2

### Template cDNA

A human type I Collagen 1A2 cDNA was obtained from the ATCC (Accession No: Y00724). Two naturally occurring polymorphisms have previously been found in Collagen 1A2 at positions 902 and 907 of the gene involving a G-->A and a T-->A nucleotide change respectively (Filie et al. 1993). Both polymorphisms occur often in the same predicted open loop structure of human Collagen 1A2 RNA. Polymorphic variants of human Collagen 1A2 were generated by PCR directed mutagenesis using a HindIII to XbaI PCR cassette. Resulting clones contained the following polymorphisms : Collagen 1A2 (A) has a G nucleotide at position 902 and a T nucleotide at position 907 (Sequence 9). In contrast human Collagen 1A2 (B) has A nucleotides at both positions 902 and 907 (Sequence 10). The site at 902 creates a ribozyme target site in Collagen 1A2 (B), that is a NUX site (900-902), but is not a ribozyme target site in Collagen 1A2 (A), in that it breaks the NUX rule - it has a G nucleotide in the X position. In contrast in Collagen 1A1 (A) there is a ribozyme target site at position 907, that is a GTC site (906-908) however this site is lost in Collagen 1A2 (B) the sequence is altered to GAC (906-908) thereby disrupting the ribozyme target site.

Ribozyme constructs Hammerhead ribozymes (termed Rz902 and Rz907) were designed to target a predicted open loop structures in the RNA from the coding region of polymorphic variants of the human Collagen 1A2 gene. Rz902 and Rz907 primers were synthesised, annealed and cloned into the HindIII and XbaI sites of pCDNA3 again allowing subsequent expression of RNA from the T7 or CMV promoters in the vector (Sequences 11 and 12). The target sites were NUX and GUX sites at positions 900-902 and 906-908 of the human type I collagen 1A2 sequence (Accession number: Y00724). Antisense flanks are underlined.
Rz902: GGTCCAGCTGATGAGTCCGTGAGGACGAAAGGACCA
Rz907: CGGCGGCTGATGAGTCCGTGAGGACGAAACCAGCA

### FIGURE LEGENDS

### Diagram 1

pBR322 was cut with MspI, radioactively labeled and run on a polyacrylamide gel to enable separation of the resulting DNA fragments. The sizes of these fragments are given in diagram 1. This DNA ladder was then used on subsequent polyacrylamide gels (4-8%) to provide an estimate of the size of the RNA products run on the gels. However there is a significant difference in mobility between DNA and RNA depending on the percentage of polyacrylamide and the gel running conditions - hence the marker provides an estimate of size of transcripts.

### Figure 1

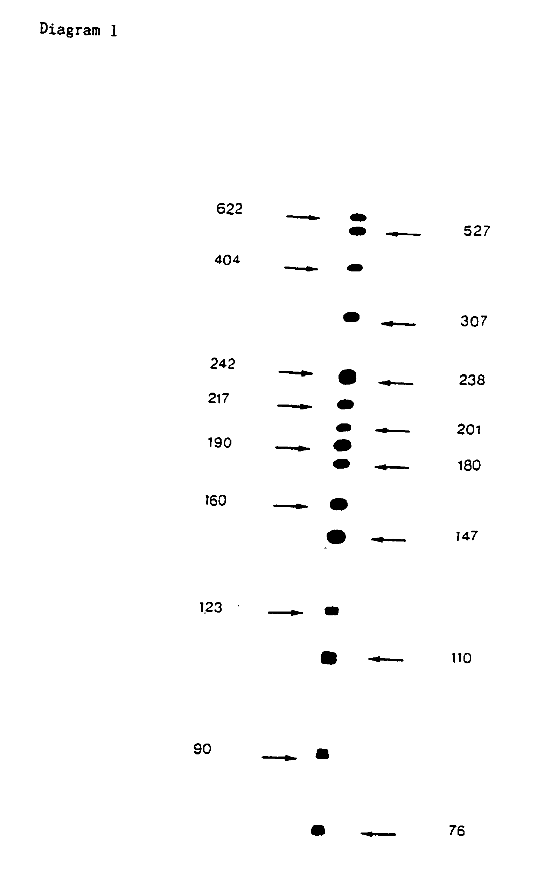

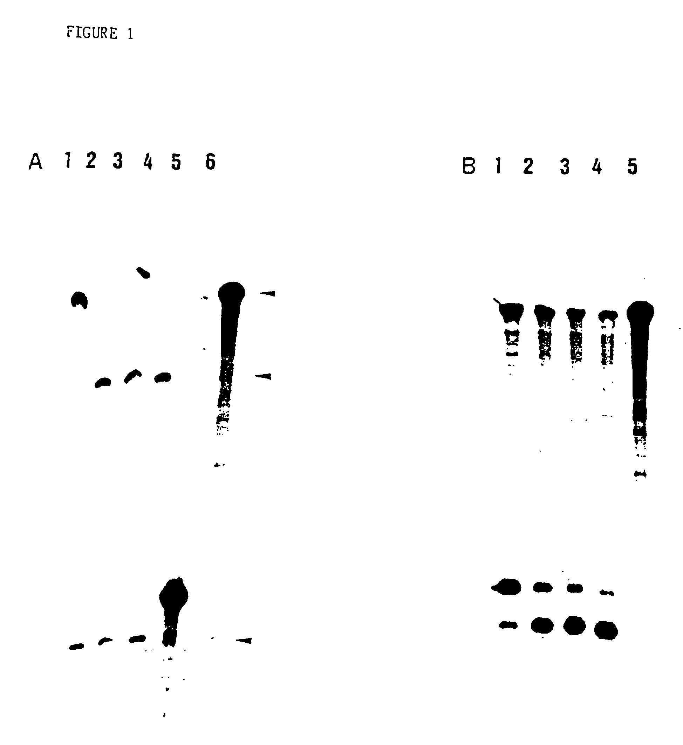

A: Human rhodopsin cDNA was expressed from the T7 promoter to the BstEII site in the coding sequence. Resulting RNA was mixed with Rz10RNA in 15mM magnesium chloride and incubated at 37°C for varying times. Lanes 1-4: Human rhodopsin RNA and Rz10RNA after incubation at 37°C with 15mM magnesium chloride for 0, 1 2 and 3 hours respectively. Sizes of the expressed RNAs and cleavage products are as expected (Table 1). Complete cleavage of human rhodopsin RNA was obtained with a small residual amount of intact RNA present at 1 hour. Cleavage products are highlighted by arrows. Lane 6 is intact unadapted human rhodopsin RNA (BstEII) alone. Lane 5 is unadapted human rhodopsin RNA (FspI) alone and refers to Figure 1B. From top to bottom, human rhodopsin RNA and the two cleavage products from this RNA are highlighted with arrows.
B: The unadapted human rhodopsin cDNA was expressed from the T7 promoter to the FspI site in the coding sequence. The adapted human rhodopsin cDNA was expressed from the T7 promoter to the BstEII site in the coding sequence. Lanes 1-4: Resulting RNAs were mixed together with Rz10 and 15mM magnesium chloride and incubated at 37°C for varying times (0, 1 , 2 and 3 hours respectively). The smaller unadapted rhodopsin transcripts were cleaved by Rz10 while the larger adapted transcripts were protected from cleavage by Rz10. Cleavage of adapted protected transcripts would have resulted in products of 564bases and 287bases - the 564bases product clearly is not present - the 287bp product is also generated by cleavage of the unadapted human rhodopsin transcripts and hence is present (FspI). After 3 hours the majority of the unadapted rhodopsin transcripts has been cleaved by Rz10. Lane 5 contains the intact adapted human rhodopsin RNA (BstEII) alone. From top to bottom adapted uncleaved human rhodopsin transcripts, residual unadapted uncleaved human rhodopsin transcripts and the larger of the cleavage products from unadapted human rhodopsin transcripts are highlighted by arrows. The smaller 22 bases cleavage product from the unadapted human rhodopsin transcripts has run off the gel.

### Figure 2

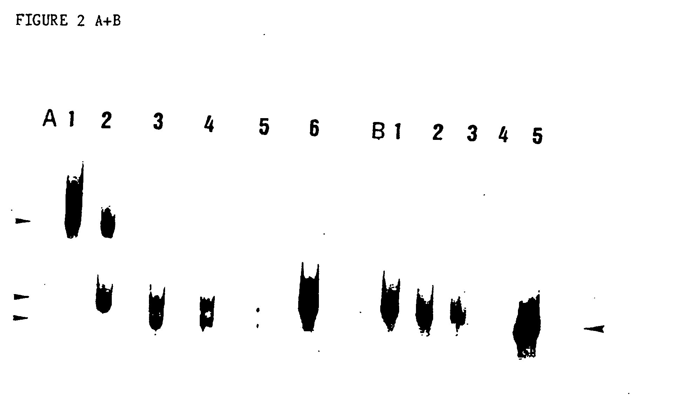

A: Unadapted and adapted human rhodopsin cDNAs were expressed from the T7 promoter to the AcyI after the coding sequence and the BstEII site in the coding sequence respectively. Sizes of expressed RNAs and cleavage products were as predicted (Table 1). Resulting RNAs were mixed together with Rz10RNA at varying magnesium chloride concentrations and incubated at 37°C for 3 hours. Lane 1 is intact unadapted human rhodopsin RNA (AcyI) alone. Lanes 2-5: Unadapted and adapted human rhodopsin RNAs and Rz10RNA after incubation at 37°C with 0, 5, 10 and 15 mM MgCl2 respectively. Almost complete cleavage of the larger unadapted human rhodopsin RNA was obtained with a small residual amount of intact RNA present at 5 mM MgCl2. In contrast the adapted human rhodopsin RNA remained intact. From top to bottom, the unadapted and adapted rhodopsin RNAs, and two cleavage products from the unadapted human rhodopsin RNA are highlighted by arrows. Lane 6 is intact adapted human rhodopsin RNA (BstEII) alone. B: The adapted human rhodopsin cDNA was expressed from the T7 promoter to the BstEII site in the coding sequence. Lanes 1-4: Resulting RNA was mixed together with Rz10 and 0, 5, 10 and 15 mM magnesium chloride and incubated at 37°C for 3 hours respectively. The adapted rhodopsin transcripts were not cleaved by Rz10. Cleavage of adapted transcripts would have resulted in cleavage products of 564bases and 287bases which clearly are not present. Lane 5: intact adapted human rhodopsin RNA (BstEII) alone. Lane 4: RNA is absent - due to a loading error or degradation. The adapted uncleaved human rhodopsin RNA is highlighted by an arrow. C: Unadapted and adapted human rhodopsin cDNAs were expressed from the T7 promoter to the AcyI after the coding sequence and the BstEII site in the coding sequence respectively. Sizes of expressed RNAs and cleavage products were as predicted (Table 1). Resulting RNAs were mixed together with Rz10RNA at varying magnesium chloride concentrations and incubated at 37°C for 3 hours. Lane 1:
DNA ladder as in Diagram 1. Lanes 2-5: Unadapted and adapted human rhodopsin RNAs and Rz10RNA after incubation at 37°C with 0, 5, 10 and 15 mM MgCl2 respectively. Almost complete cleavage of the larger unadapted human rhodopsin RNA was obtained with a small residual amount of intact RNA present at 5 and 10 mM MgCl2. In contrast the adapted human rhodopsin RNA remained intact. Lane 6: Adapted human rhodopsin RNA (BstEII) alone. Lane 7: Unadapted human rhodopsin RNA (AcyI) alone. Lane 8: DNA ladder as in Diagram 1. From top to bottom, the unadapted and adapted rhodopsin RNAs, and two cleavage products from the unadapted human rhodopsin RNA are highlighted by arrows. Separation of the adapted human rhodopsin RNA (851bases) and the larger of the cleavage products from the unadapted RNA (896bases) is incomplete in this gel (further runnning of the gel would be required to achieve separation) - however the separation of these two RNAs is demonstrated in Figure 2A.

### Figure 3

The mutant (Pro23Leu) human rhodopsin cDNA was expressed from the T7 promoter to the BstEII in the coding sequence. Likewise the Rz20 clone was expressed to the XbaI site. Resulting RNAs were mixed together with 5mM magnesium chloride concentrations at 37°C for varying times. Sizes of expressed RNAs and cleavage products were as predicted (Table 1). Lane 1: DNA ladder as in Diagram 1. Lanes 2: Pro23Leu human rhodopsin RNA alone. Lanes 3-7 Pro23Leu human rhodopsin RNAand Rz20RNA after incubation at 37°C with 10 mM MgCl2 for 0mins, 30 mins, 1 hr, 2hrs and 5hrs respectively. Almost complete cleavage of mutant rhodopsin transcripts was obtained with a residual amount of intact RNA left even after 5 hours. Lane 8: DNA ladder as in Diagram 1. From top to bottom, the uncleaved RNA and the two cleavage products from the mutant human rhodopsin RNA are highlighted by arrows.

### Figure 4

The mutant (Pro23Leu) human rhodopsin cDNA was expressed from the T7 promoter to the BstEII in the coding sequence. Likewise the Rz10 clone was expressed to the XbaI site. Resulting RNAs were mixed together with 10mM magnesium chloride concentrations at 37°C for varying times. Sizes of expressed RNAs and cleavage products were as predicted (Table 1). Lane 1: DNA ladder as in Diagram 1. Lanes 2: Pro23Leu human rhodopsin RNA alone. Lanes 3-7 Pro23Leu human rhodopsin RNAand Rz10RNA after incubation at 37°C with 10 mM MgCl2 for 0mins, 30 mins, 1 hr, 2hrs and 5hrs respectively. Almost complete cleavage of mutant human rhodopsin RNA was obtained with a residual amount of intact RNA remaining even after 5 hours (Lane 7). Lane 8: DNA ladder as in Diagram 1. From top to bottom, intact mutant rhodopsin RNA and the two cleavage products from the mutant human rhodopsin RNA are highlighted by arrows.

### Figure 5

A: The human collagen 1A1 cDNA clones containing the T allele of the polymorphism at 3210 was expressed from the T7 promoter to the XbaI site in the vector. Resulting RNA was mixed together with RzPolCol1A1 at various magnesium chloride concentrations and incubated at 37°C for 3 hours. Lane 1: intact RNA from the human collagen 1A1 T allele alone. Lanes 2-5: Human collagen 1A1 T allele RNA and RzPolCol1A1 incubated with 0, 5, 10, 15 mM MgCl2 at 37°C for 3 hours. RNA transcripts are cleaved efficiently by RzPolCol1A1 - a residual amount of RNA remained at 5mM MgCl2. Lane 6: DNA ladder as in Diagram 1. From top to bottom, intact T allele RNA and two cleavage products from this RNA are highlighted by arrows. B: The human collagen 1A1 cDNA clones containing the C allele of the polymorphism at 3210 was expressed from the T7 promoter to the XbaI site in the vector. Resulting RNA was mixed together with RzPolCol1A1 at various magnesium chloride concentrations and incubated at 37°C for 3 hours. Lane 1: DNA ladder as in Diagram 1. Lane 2: intact RNA from the human collagen 1A1 C allele alone. Lanes 3-6: Human collagen 1A1 C allele RNA and RzPolCol1A1 incubated with 0, 5, 10, 15 mM MgCl2 at 37°C for 3 hours. RNA transcripts were not cleaved by RzPolCollA1 - RNA remained intact over a range of MgCl2 concentrations (highlighted by an arrow). No cleavage products were observed in any of the lanes. Lane 6 has significantly less RNA due to a loading error. Lane 7: DNA ladder as in Diagram 1.

### Figure 6

The human collagen 1A1 cDNA clones containing the T allele of the polymorphism at 3210 was expressed from the T7 promoter to the XbaI site in the vector. Resulting RNA was mixed together with RzPolCol1A1 at 5mM magnesium chloride concentrations and incubated at 37°C for varying times. Lane 1: DNA ladder as in Diagram 1. Lane 2: intact RNA from the human collagen 1A1 T allele alone. Lanes 3-7: Human collagen 1A1 T allele RNA and RzPolCol1A1 incubated with10mM MgCl2 at 37°C for 0, 30 mins, 1hour, 2hours and 5hours respectively. Transcripts are cleaved by RzPolCol1A1 immediately upon addition of MgCl2. From top to bottom, the T allele RNA and cleavage products are highlighted by arrows. Lane 8: DNA ladder as in Diagram 1.

### Figure 7

The human collagen 1A1 cDNA clones containing the C allele of the polymorphism at 3210 was expressed from the T7 promoter to the XbaI site in the vector. Resulting RNA was mixed together with RzPolCol1A1 with 5mM magnesium chloride and incubated at 37°C for varying times. Lane 1: DNA ladder as in Diagram 1. Lane 2: intact RNA from the human collagen 1Al C allele alone. Lanes 3-7: Human collagen 1A1 C allele RNA and RzPolCol1A1 incubated with 10mM MgCl2 at 37°C for 0, 30 mins, 1hour, 2hours and 5hours respectively. RNA transcripts are not cleaved by RzPolCol1A1 even after 5 hours - no cleavage products were observed. The intact RNA from the C allele is highlighted by an arrow. Lane 8: DNA ladder as in Diagram 1.

### Figure 8

A: The human collagen 1A2 cDNA clones containing the A and T alleles of the polymorphism at position 907 were expressed from the T7 promoter to the MvnI and XbaI sites in the insert and vector respectively. Resulting RNAs were mixed together with Rz907 and various MgCl2 concentrations and incubated at 37°C for 3 hours. Lane 1: intact RNA from the human collagen 1A2 (B) containing the A allele of the 907 polymorphism. Lane 2: intact RNA from the human collagen 1A2 (A) containing the T allele of the 907 polymorphism. Lanes 3-5: Human collagen 1A2 (A) and (B) representing the A and T allele RNAs and Rz907 incubated with 0, 5, and 10 mM MgCl2 at 37°C for 3 hours. RNA transcripts from the T allele containing the 907 target site are cleaved by Rz90 upon addition of divalent ions - almost complete cleavage is obtained at 10mM MgCl2 with a residual amount of transcript from the T allele remaining (Lane 5). In contrast transcripts expressed from the A allele (which are smaller in size to distinguish between the A (MvnI) and T (XbaI) alleles) were not cleaved by Rz907 - no cleavage products were observed. From top to bottom, RNA from the T allele, the A allele and the two cleavage products from the T allele are highlighted by arrows. Lane 6: DNA ladder as in Diagram 1.

B: The human collagen 1A2 cDNA (A) + (B) clones containing the A and T alleles of the polymorphism at 907 were expressed from the T7 promoter to the MvnI and XbaI sites in the insert and vector respectively. Resulting RNAs were mixed together with Rz907 and 10mM magnesium chloride and incubated at 37°C for varying times. Lane 1: DNA ladder as in Diagram 1. Lane 2: intact RNA from the human collagen 1A2 (B) with the A allele of the 907 polymorphism. Lane 3: intact RNA from the human collagen 1A2 (A) with the T allele of the 907 polymorphism. Lanes 4-9: Human collagen 1A2 A and T allele RNA and Rz907 incubated with10mM MgCl2 at 37°C for 0, 30 mins, 1hour, 2hours, 3 hours and 5hours respectively. RNA transcripts from the T allele containing the 907 target site are cleaved by Rz905 - complete cleavage is obtained after 5 hours. In contrast transcripts expressed from the A allele (which are smaller in size to distinguish between the A (MvnI) and T (XbaI) alleles) were not cleaved by Rz907 - no cleavage products were observed. From top to bottom, RNA from the T allele, the A allele and the two cleavage products from the T allele are highlighted.

### Figure 9

A: The human collagen 1A2 cDNA (A) and (B) clones containing the G and A alleles of the polymorphism at position 902 were expressed from the T7 promoter to the MvnI and XbaI sites in the insert and vector respectively. Resulting RNAs were mixed together with Rz902 and various magnesium chloride concentrations and incubated at 37°C for 3 hours. Lane 1: DNA ladder as in Diagram 1. Lane 2: intact RNA from the human collagen 1A2 (B) with A allele of the 902 polymorphism Lane 3: intact RNA from the human collagen 1A2 (A) with the G allele of the 902 polymorphism. Lanes 4-7: Human collagen 1A2 A and G allele RNA and Rz902 incubated with 0, 5, 10 and 15 mM MgCl2 at 37°C for 3 hours. RNA transcripts from the B allele containing the 902 target site are cleaved by Rz902 upon addition of divalent ions - the cleavage obtained with Rz902 is not very efficient. In contrast transcripts expressed from the G allele (which are smaller in size to distinguish between the G (MvnI) and A (XbaI) alleles) were not cleaved at all by Rz902 - no cleavage products were observed. From top to bottom, RNA from the A allele, the B allele and the two cleavage products from the A allele are highlighted. Lane 8: DNA ladder as in Diagram 1.

### RESULTS

Human rhodopsin and human collagen 1A1 and 1A2 cDNA clones representing specific polymorphic variants of these genes were expressed in vitro. Ribozymes targeting specific alleles of the human rhodopsin and collagen 1A1 and 1A2 cDNAs were also expressed in vitro. cDNA clones were cut with various restriction enzymes resulting in the production of differently sized transcripts after expression. This aided in differentiating between RNAs expressed from cDNAs representing different alleles of polymorphisms in rhodopsin and collagen 1A1 and 1A2. Restriction enzymes used to cut each clone, sizes of resulting transcripts and predicted sizes of products after cleavage by target ribozymes are given below in Table 1. Exact sizes of expression products may vary by a few bases from that estimated as there is some ambiguity about the specific base at which transcription starts (using the T7 promoter) in pCDNA3.

### Example 1

### A: Human Rhodopsin

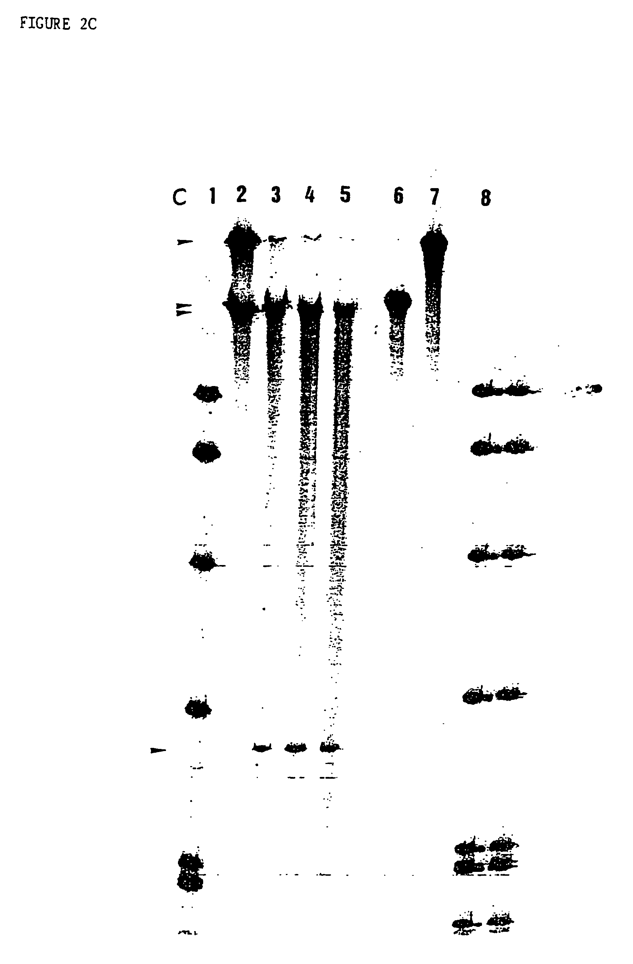

The unadapted human rhodopsin cDNA and the human rhodopsin cDNA with a single nucleotide substitution in the coding sequence were cut with BstEII and expressed in vitro. The single base change occurs at the third base position of the codon (at position 477) and therefore does not alter the amino acid coded by this triplet. The polymorphism is artificially derived, however, it mirrors naturally occurring polymorphisms in many genes which contain single nucleotide alterations that are silent. The Rz10 clone was cut with XbaI and expressed in vitro. Resulting ribozyme and human rhodopsin RNAs were mixed with varying concentrations of MgCl2 to optimise cleavage of template RNA by Rz10. A profile of human rhodopsin RNA cleavage by Rz10 over time is given in Figure 1A. The MgC12 curve profile used to test if adapted human rhodopsin transcripts could be cleaved by Rz10 is given in Figure 2B. Unadapted and adapted human rhodopsin cDNAs were cut with FspI and BstEII respectively, expressed and mixed together with Rz10 RNA to test for cleavage (Figure 1B) over time. Likewise, unadapted and adapted human rhodopsin cDNAs were cut with AcyI and BstEII respectively, both were expressed in vitro and resulting transcripts mixed with Rz10 RNA at varying MgCl2 concentrations to test for cleavage (Figure 2A, 2C). In all cases expressed RNAs were the predicted size. Similarly in all cases unadapted transcripts were cleaved into products of the predicted size. Cleavage of unadapted human rhodopsin RNA was almost complete - little residual uncleaved RNA remained. In all cases adapted human rhodopsin RNAs with a single base change at a silent site remained intact, that is, it was not cleaved by Rz10. Clearly, transcripts from one allele of this artificial polymorphism are cleaved by Rz10 while transcripts from the other allele are protected from cleavage. It is worth noting that AcyI enzyme cuts after the stop codon and therefore the resulting RNA includes the complete coding sequence of the gene.

### B: Human Rhodopsin

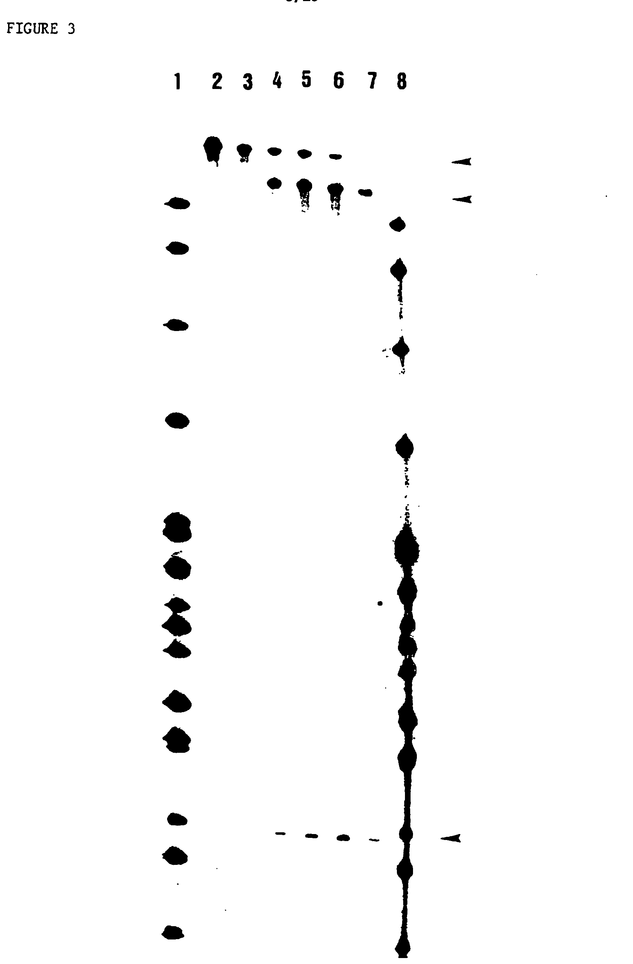

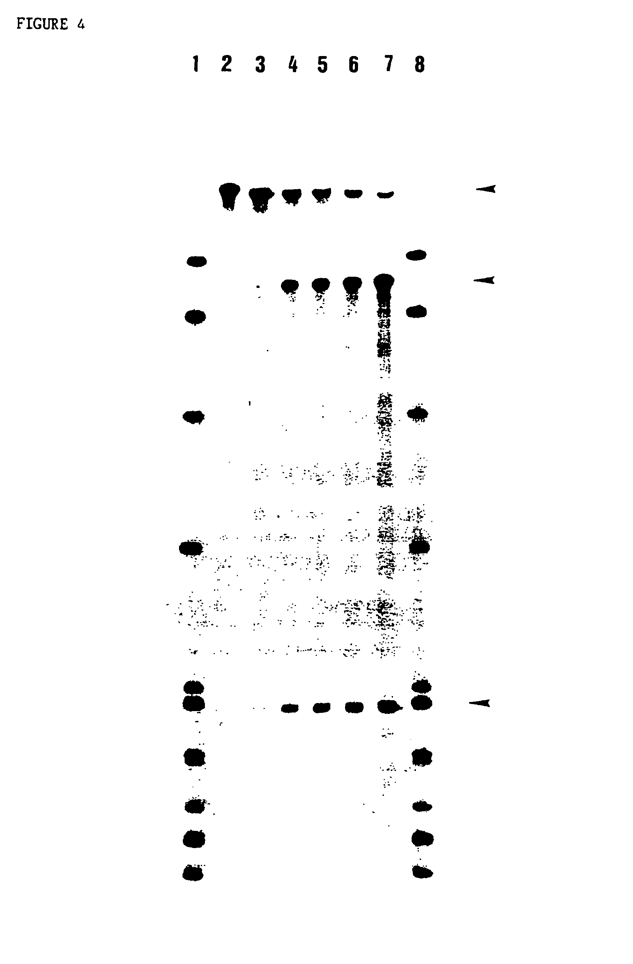

Rz20 was cut with XbaI and expressed in vitro. Similarly the rhodopsin cDNA containing a Pro23Leu mutation was cut with BstEII and expressed in vitro. Resulting RNAs were mixed and incubated with varying concentrations of MgCl2. Rz20 was designed to elicit mutation specific cleavage of transcripts containing a Pro23Leu rhodopsin mutation. All expressed products and cleavage products were the correct size. Figure 3 demonstrates mutation specific cleavage of the mutant RNA over time incublated at 37°C with 10mMMgCl₂. Cleavage of mutant rhodopsin transcripts by Rz10 which targets a ribozyme cleavage site 3' of the site of the Pro23Leu mutation in one allele of an artificially derived polymorphism in rhodopsin coding sequence was explored. The mutant rhodopsin cDNA and Rz10 clones were cut with BstEII and XbaI respectively and expressed in vitro. Resulting RNAs were mixed and incubated with 10mMMgCl2 for varying times (Figure 4). All expressed products and cleavage products were the correct size. Rz10 cleaved mutant rhodopsin transcripts when the mutation was on the same allele of the polymorphism targeted by Rz10. Using an artificially derived allelic variant around the Rz10 cleavage site we demonstrated in Example 1A that transcripts from the artificial allele remain intact due to absence of the Rz10 target site (Figures 1B, 2A and 2B). Hence Rz10 could be used to cleave mutant transcripts in a manner independent of the disease mutation itself (that is, using a polymorphism) while wild type transcripts from the alternative allele (in this case artificially derived to exemplify the process for rhodopsin) would remain intact and therefore could supply the wild type protein.

### Example 2

### Human Collagen 1A1

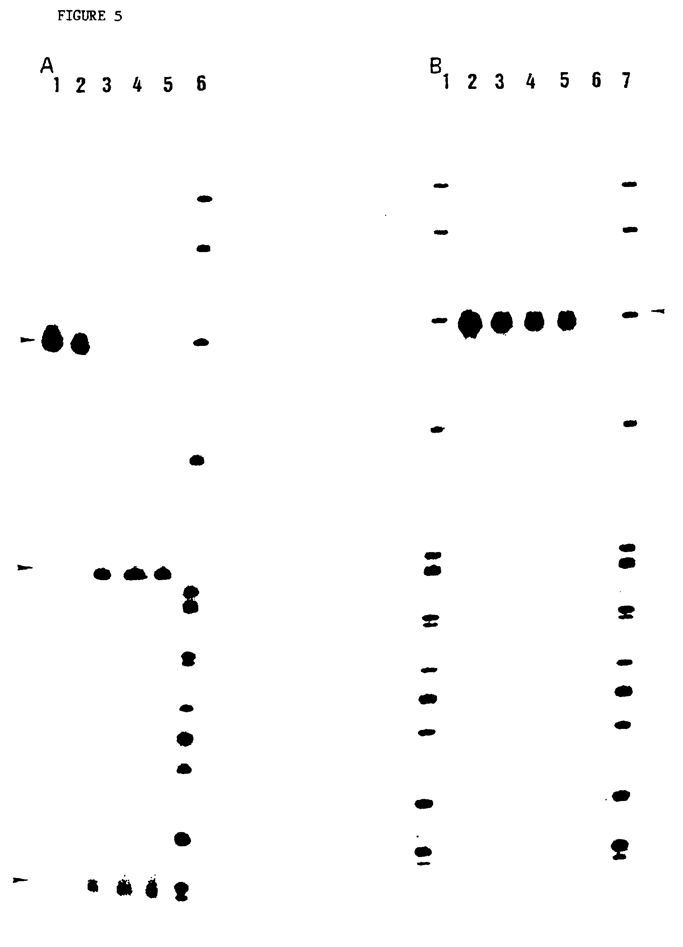

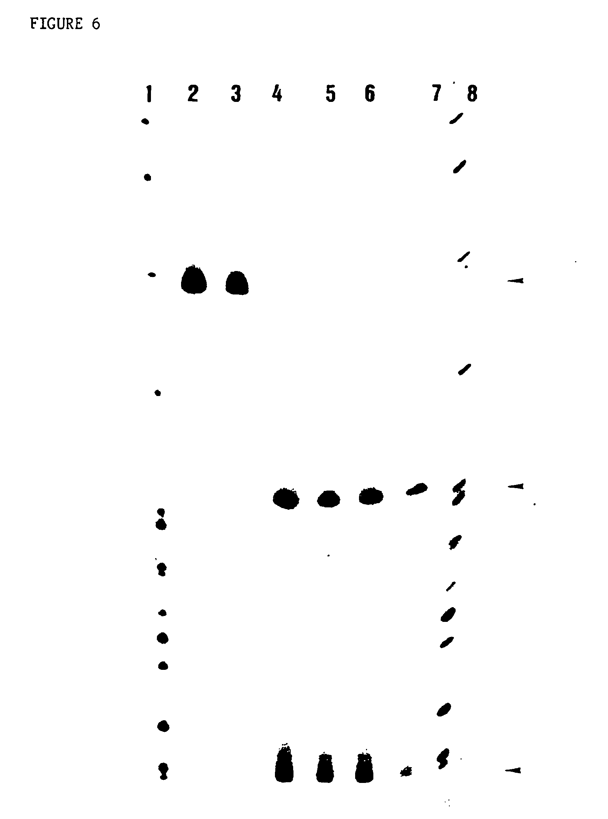

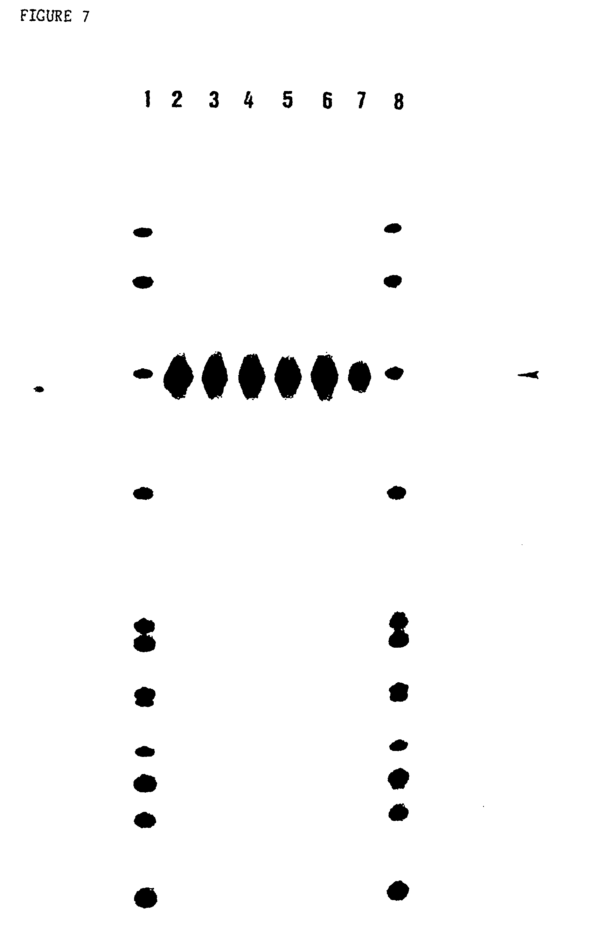

RzPolCol1A1 clones targeting a polymorphic site in human collagen 1A1 sequence were cut with XbaI and expressed in vitro. The human collagen 1A1 cDNA clones (A and B) containing the two allelic forms of a naturally occurring polymorphism (T/C) in the 3'UTR of the gene at position 3210 of the sequence were cut with XbaI, expressed in vitro and both RNAs mixed separately with RzPolCol1A1 RNA to test for cleavage. RNAs were mixed with varying concentrations of MgCl₂ to optimise cleavage of RNAs by RzPolCol1A1 (Figure 5). Notably, the majority of the RNA transcripts from human collagen 1A1 (A) which has a T nucleotide at position 3210 and therefore contains a ribozyme cleavage site GTC (3209-3211) were cleaved while transcripts from the other allele (Collagen 1A1 (B)) which has a C nucleotide at this position remained intact (Figure 5). Cleavage of collagen 1A1 transcripts over time in 10mM MgCl2 was assessed for the T allele of the polymorphism (Figure 6) and the C allele of the polymorphism (Figure 7) at position 3210.

### Example 3

### Human Collagen 1A2

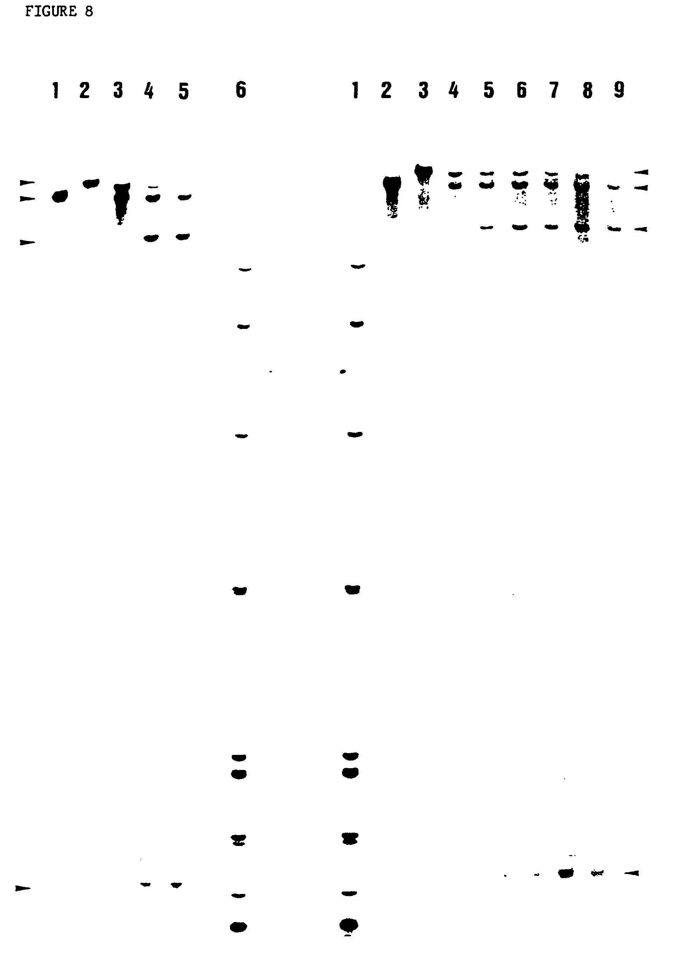

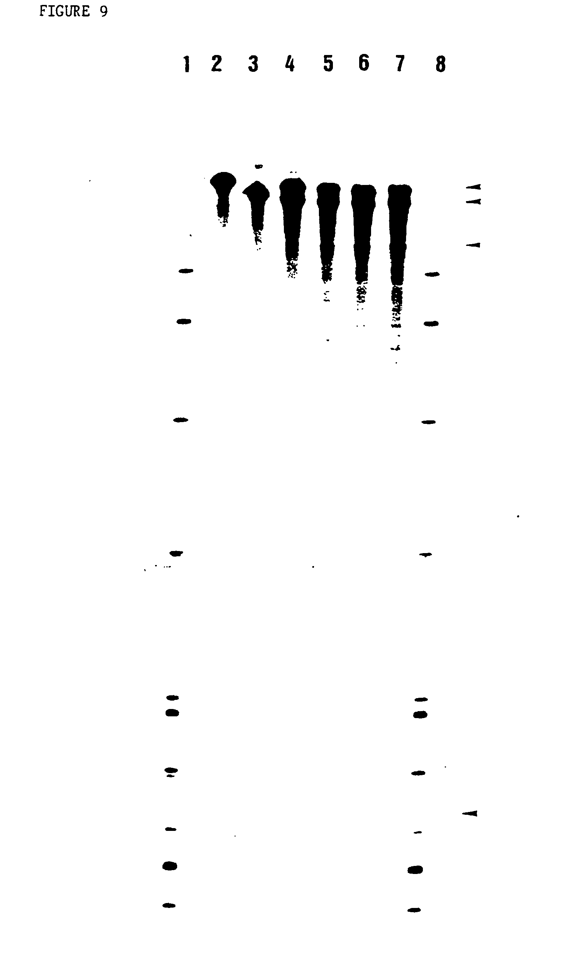

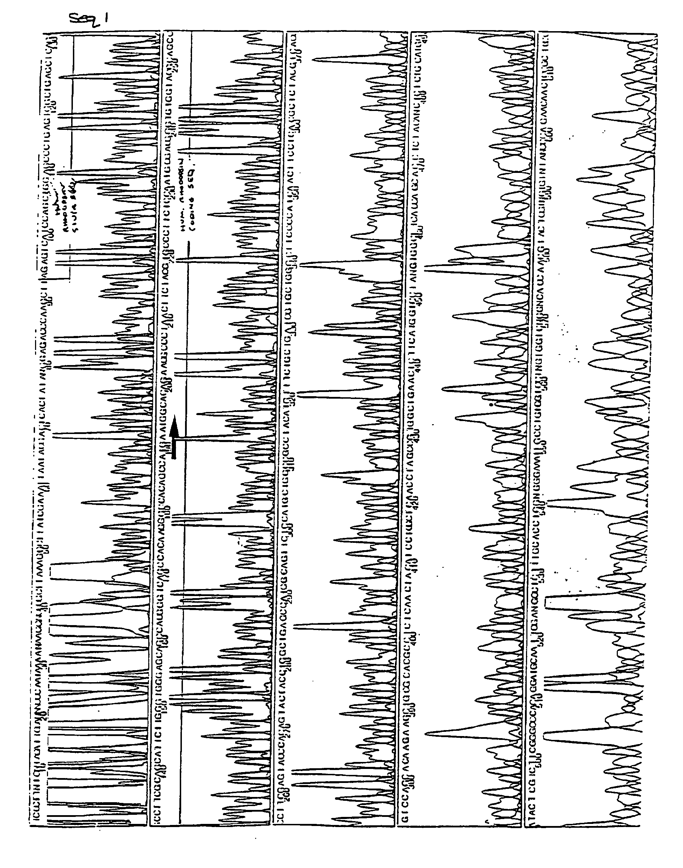

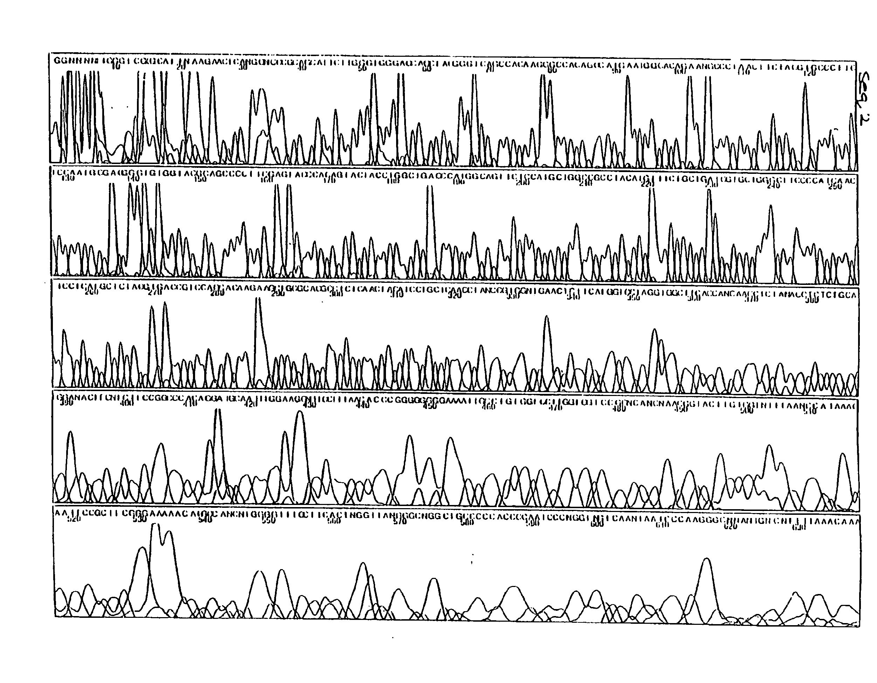

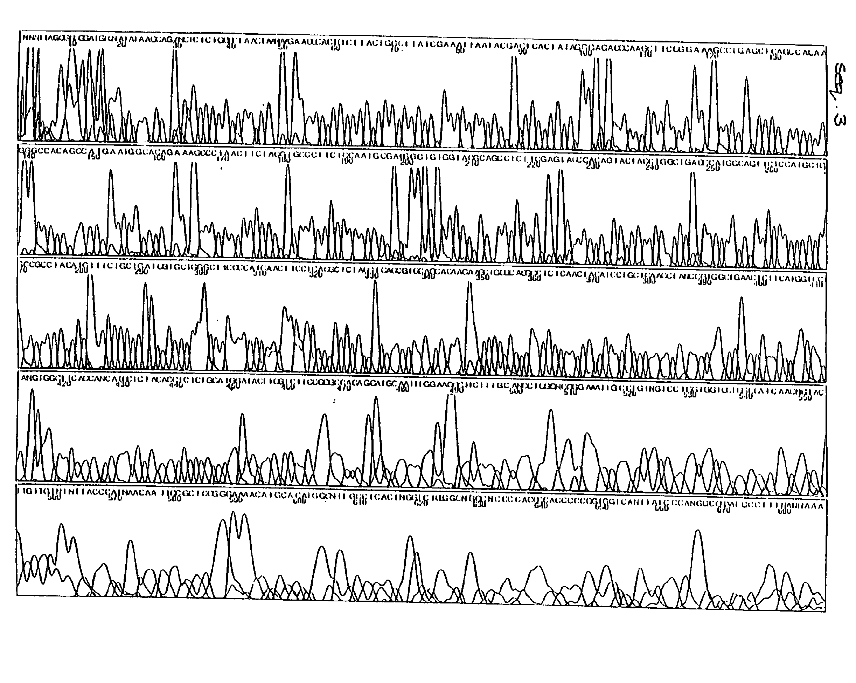

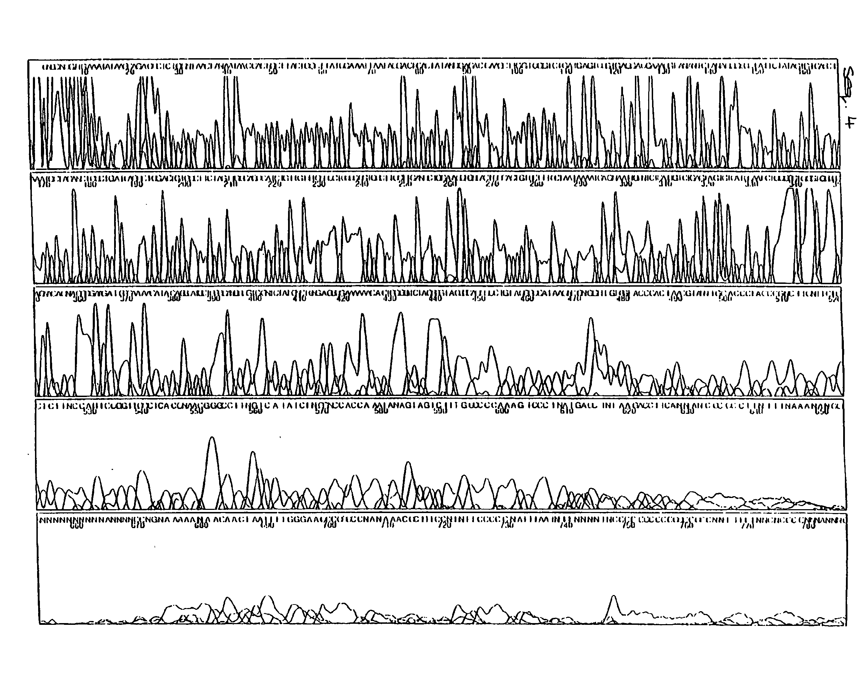

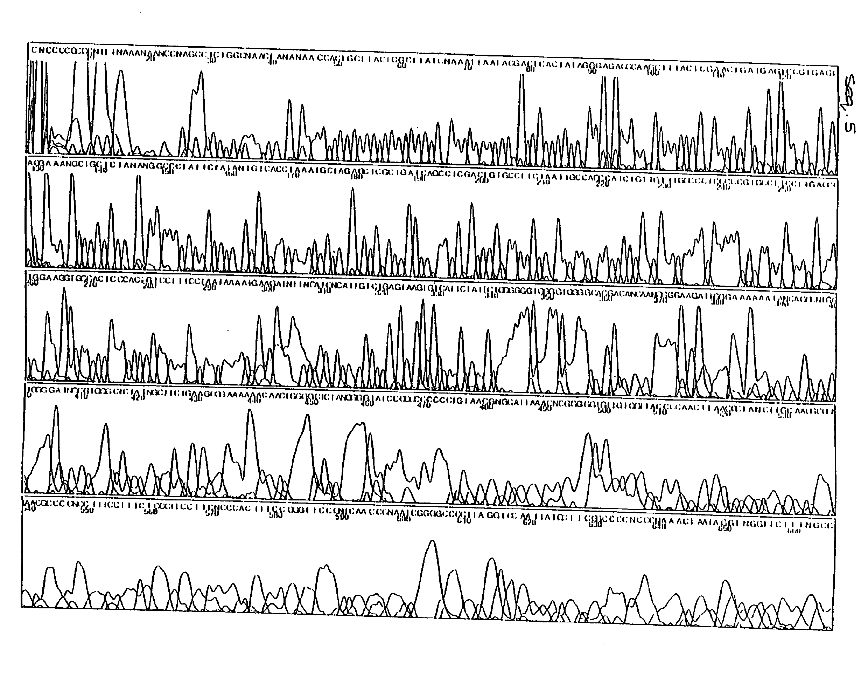

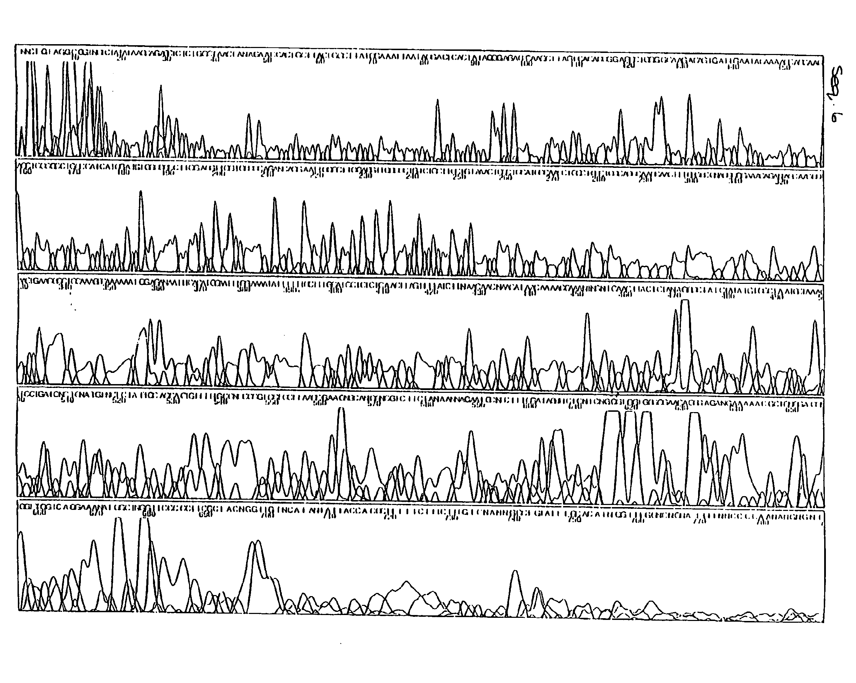

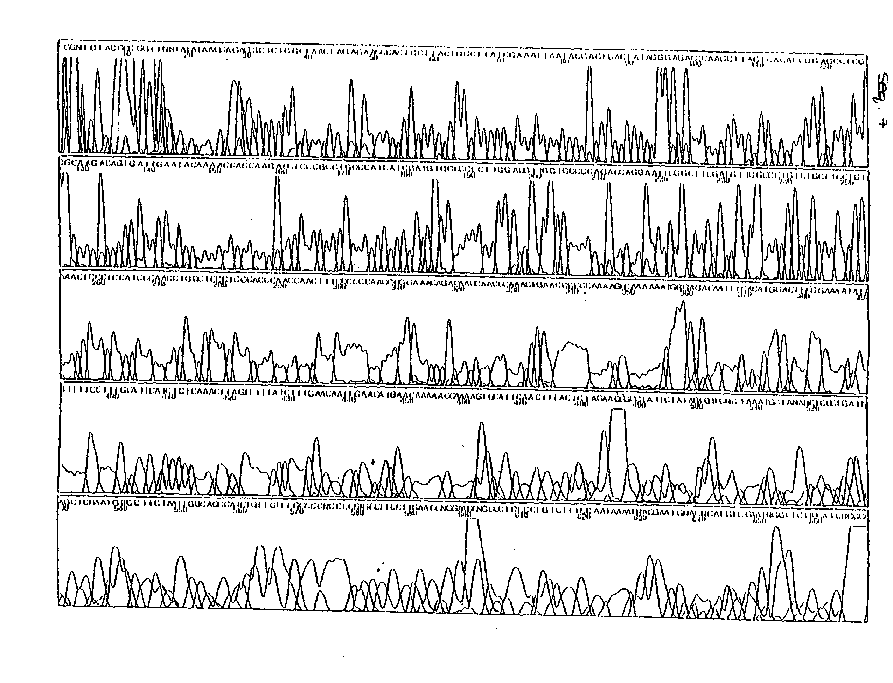

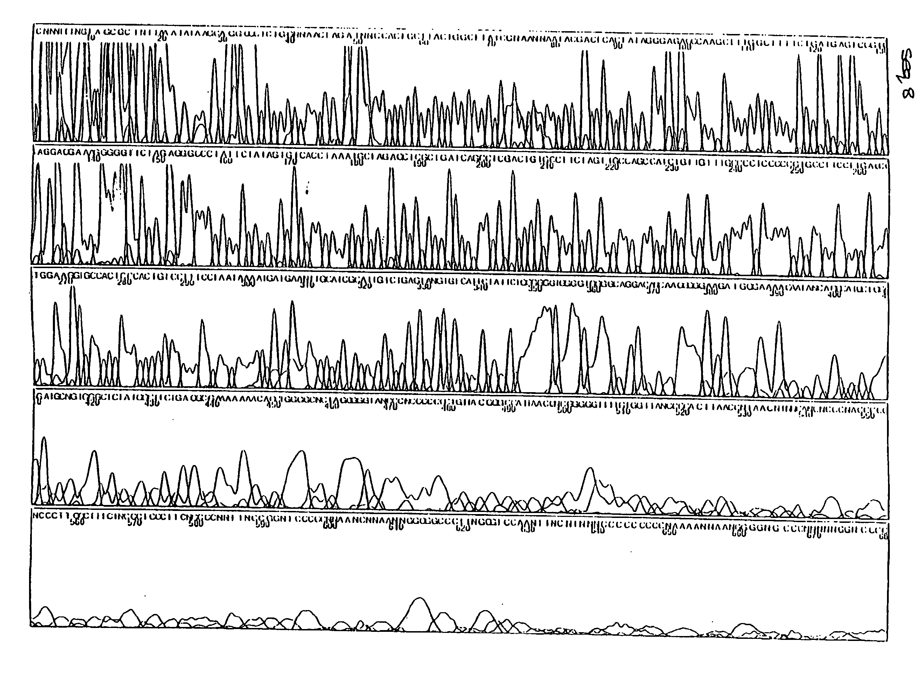

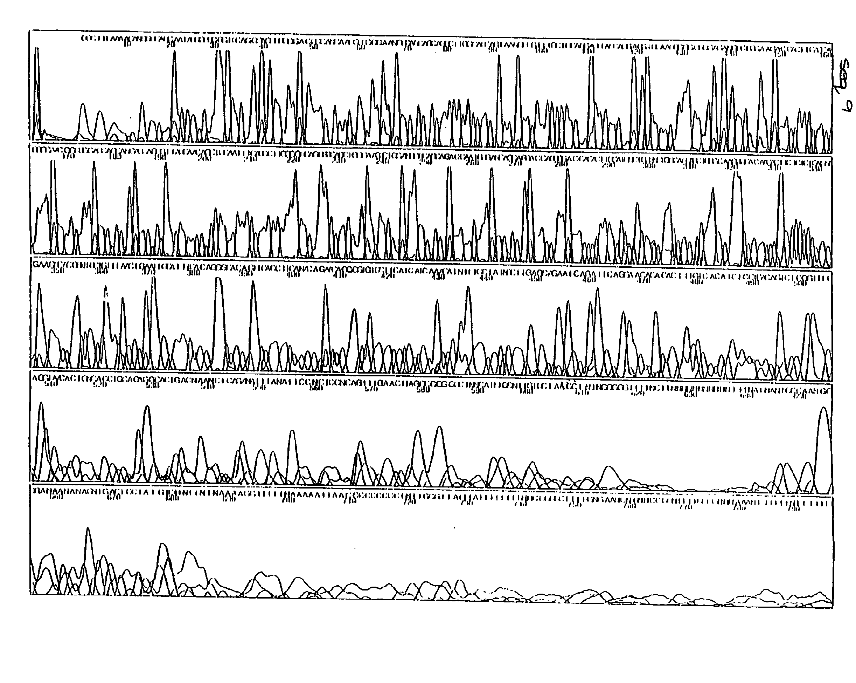

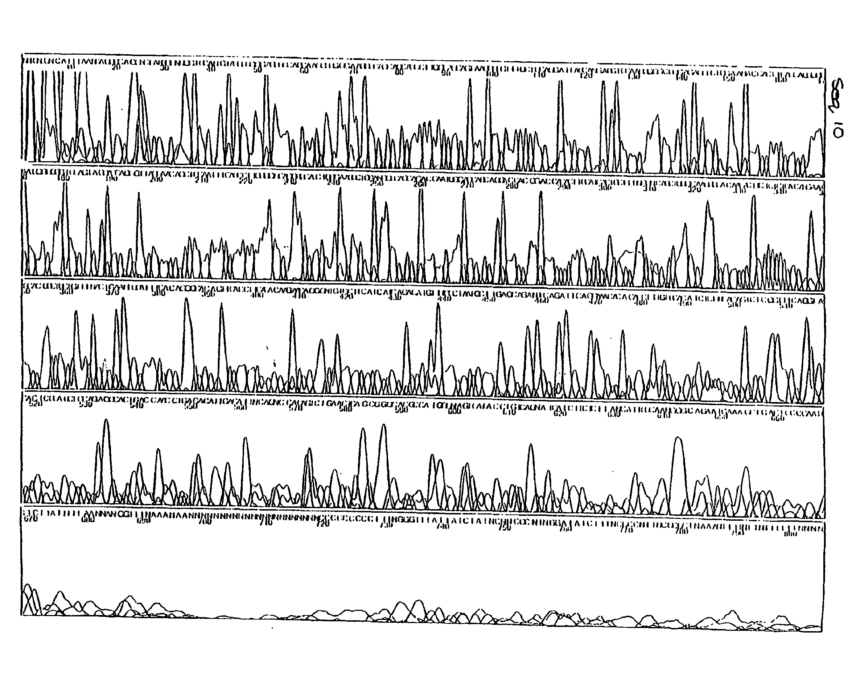

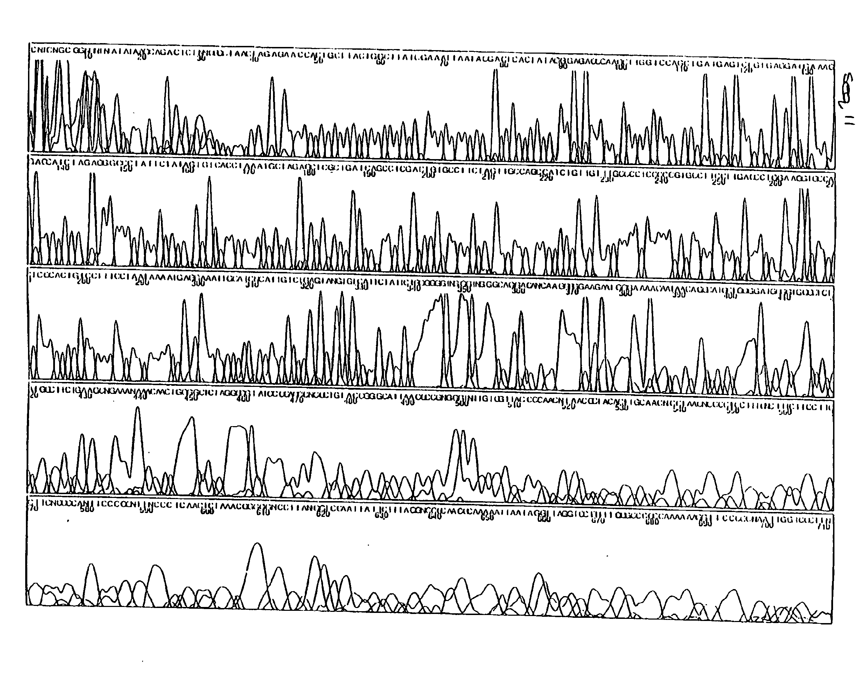

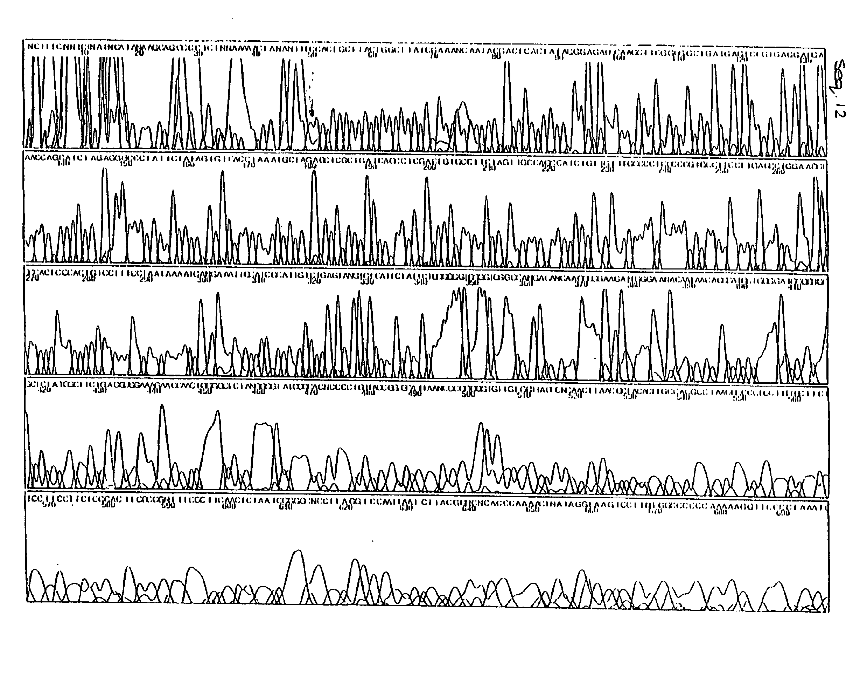

Rz902 and Rz907 clones targeting a polymorphic site in human collagen 1A2 sequence were cut with XbaI and expressed in vitro. The human collagen 1A2 cDNA clones (A and B) containing two allelic forms of two polymorphisms in the coding sequence of the gene at positions 902 and 907 of the sequence were both cut with both XbaI and MvnI, expressed in vitro and RNAs mixed together with Rz902 or Rz907 RNA to test for cleavage of transcripts by these ribozymes. All expressed transcripts were of the predicted sizes. RNAs were mixed with varying concentrations of MgCl₂ to optimise cleavage of RNAs by Rz902 and Rz907 (Figures 8 and 9). Notably the majority of the RNA transcripts from human collagen 1A2 (A) which has a G nucleotide at position 902 and a T nucleotide at position 907 is cleaved by Rz907 (Figure 8). Cleavage products were the correct size. In contrast human collagen 1A2 (A) transcripts were not cleaved by Rz902 (Figure 9). This allelic form of the gene has a ribozyme cleavage site at 907 but does not have a cleavage site at position 902. Notably the situation is reversed with transcripts from human collagen 1A2 (B) where in this allelic form of the gene due the nature of the polymorphisms present there is a ribozyme cleavage site at position 902 but the site which in the other allelic form of the gene was at position 907 has been lost. Transcripts from human collagen 1A2 (B) were cleaved specifically by Rz902 - cleavage products were the correct size (Figure 9). In contrast, transcripts from this allelic form of the gene were protected from cleavage by Rz907 due to the alteration in the sequence around the ribozyme cleavage site (Figure 8). Cleavage of collagen 1A2 (B) by Rz902 was less efficient than cleavage of collagen 1A2 (A) by Rz907. This is consistent with 2-D predictions of RNA open loop structures for the two polymorphisms - in the allele containing the Rz907 ribozyme cleavage site, the target site is found more consistently in an open loop structure when compared to the Rz902 cleavage site. However, these two polymorphisms which are in strong linkage disequilibrium with each other (separated by 6 bases only) and which are often found in the same open loop structure of the transcript clearly demonstrate the feasibility and utility of polymorphisms in directing suppression effectors to different alleles of genes, in this case the human collagen 1A2 gene.

**TABLE 1**

| | Restriction Enzyme | RNA Size | Cleavage Products |
|---|---|---|---|
| Example 1 | | | |
| Human rhodopsin | BstEII | ~851bases | 287+564 bases |
| | AcyI | ~1183bases | 287+896 bases |
| | FspI | ~309bases | 287+22 |
| Human rhodopsin | | | |
| artificial | | | |
| polymorphism | BstEII | ~851bases | |
| Human rhodopsin | | | |
| Pro-Leu | BstEII | ~851bases | 170+681(Rz20) |
| Human rhodopsin | | | |
| Pro-Leu | BstEII | ~851bases | 287+564(Rz10) |
| Rz10 | XbaI | ~52bases | |
| Rz20 | XbaI | ~52bases | |
| (Table 1; Sequences 1-5 ; Figures 1-4) | | | |
| Example 2 | | | |
| Human Collagen | | | |
| 1A1 (A) | XbaI | ~381bases | 245+136bases |
| Human Collagen | | | |
| 1A1 (B) | XbaI | ~381bases | 245+136bases |
| RzPolCol 1A1 | XbaI | ~52bases | |
| (Table 1; Sequences 6-8 ; Figures 5-7) | | | |
| Example 3 | | | |
| Human Collagen | | | |
| 1A2 (A) -Rz907 | XbaI | ~888bases | 689+199bases |
| Human Collagen | | | |
| 1A2 (B) | MvnI | ~837bases | |
| Human Collagen | | | |
| 1A2 (A) | MvnI | ~837bases | |
| Human Collagen | | | |
| 1A2 (B) -Rz902 | XbaI | ~888bases | 683+205bases |
| Rz902 | XbaI | ~52bases | |
| Rz907 | XbaI | ~52bases | |
| (Table 1; Sequences 9-12; Figures 8 and 9) | | | |
| (RNA sizes are estimates) | | | |

**TABLE 2**

| | | | |
|---|---|---|---|
| A: Listing of some polymorphisms (silent/non-silent) in rhodopsin, peripherin and collagen 1A1 and 1A2 genes. The polymorphisms used in the invention are listed here - however many other polymorphisms have been characterised in the collagen 1A1 and 1A2 genes. A 38 base pair polymorphism in Collagen 1A2 is also listed. | | | |

| Rhodopsin | Peripherin | Collagen 1A1 | Collagen 1A2 |
|---|---|---|---|
| Gly 120 Gly | C558T | T(0.28)3210C (0.72) | A902G |
| Ala 173 Ala | Glu 304 Gln | | T908A |
| | Lys 310 Arg | | 38bp insert. (Dalgleish 1986) |
| | Gly 338 Asp | | |
| B: Rhodopsin mutations tested to assess if the predicted open loop RNA structure containing the Rz10 target site (475-477) remains intact in mutant transcripts. | | | |

| Rhodpsin mutation | | RNA open loop targeted by Rz10 | |
|---|---|---|---|
| Pro 23 Leu | | Intact | |
| Gly 51 Val | | Intact | |
| Thr 94 IIe | | Intact | |
| Gly 188 Arg | | Intact | |
| Met 207 Arg | | Intact | |
| IIe del 255 | | Intact | |

### DISCUSSION

In the examples outlined above, RNA was expressed from cDNAs coding for three different proteins: human rhodopsin and human type I collagen 1A1 and 1A2. Moreover, cDNA templates utilised in the invention coded for specific allelic variants of each of these three genes. In the case of rhodopsin this polymorphism is artificially derived to exemplify the invention and the potential use of the invention for retinopathies such as adRP. In contrast, for the human collagen 1A1 and 1A2 genes three separate naturally occurring polymorphisms have been used to demonstrate the invention and the potential use of the invention for disorders such as OI. The suppression effectors of choice in the invention have been hammerhead ribozymes with antisense flanks to define sequence specificity. Hammerhead ribozymes require NUX cleavage sites in open loop structures of RNA. Notably, other suppression effectors could be utilised in the invention and would lead to a more flexible choice of polymorphic target sequences for suppression. Transcripts expressed from individual allelic variants of all three genes have been significantly attacked in vitro using suppression effectors directed towards one single allelic form of the gene. In all three examples the ribozymes directed to polymorphic sites were successful in cleaving target RNAs from one allele in the predicted manner. Antisense targeting sequences surrounding the polymorphisms were used successfully to elicit binding and clearage of target RNAs in a sequence specific manner. Additionally, transcripts from an alternative allele of each of the genes tested were protected fully from cleavage by ribozymes designed to target a different allele.

The utility of individual polymorphisms to suppress one allele of a gene carrying a deleterious mutation will depend in part on the frequency of the polymorphism in a given population. In order to distinguish between two alleles of a gene in a manner which is independent of the disease mutation an individual would have to be heterozygous for the polymorphism. The proportion of individuals who will be heterozygous for a particular polymorphism will depend on the allele frequencies of the polymorphism in the population being assessed. For example, approximately 40% of individuals tested were heterozygous for collagen 1A1 3210 polymorphism. To increase the number of individuals that could be treated using suppression effectors directed to polymorphisms and in addition to increase the efficiency of suppression, multiple polymorphisms within a gene could be used when necessary.

The utility of an individual ribozyme designed to target an NUX site in an open loop structure of transcripts from one allele of a gene will depend in part on the robustness of the RNA open loop structure when various deleterious mutations are also present in the transcript. To evaluate this we analysed RNAPlotFold data for six different adRP causing mutations in the rhodopsin gene. For each of these the large RNA open loop structure which is targeted by Rz10 was maintained in the mutant transcripts (Table 2). This is clearly demonstrated in example 1B (Figure 3) using a Pro23Leu rhodopsin mutation. Rz10 clearly cleaves the mutant transcript effectively in vitro.

In some cases it is possible that lowering RNA levels may often lead to a parallel lowering of protein levels however this is not always the case. In some situations mechanisms may prevent a significant decrease in protein levels despite a substantial decrease in levels of RNA. However in many instances suppression at the RNA level has been shown to be effective (see prior art). In some cases it is thought that ribozymes elicit suppression not only by cleavage of RNA but also by an antisense effect due to the antisense arms in the ribozyme surrounding the catalytic core.

In the three examples provided ribozymes were designed to cleave single alleles at a polymorphic site. In one example, Collagen 1A2, two ribozymes were used to target two different polymorphic sites located 6 bases apart often in the same open loop structure in the predicted 2-D conformations of the transcripts - one ribozyme targets one allele of Collagen 1A2 while the second ribozyme targets the alternative allele. If necessary, multiple polymorphisms within or close to a gene targeted towards the same allele could be used to achieve efficient and specific suppression of an individual allele. For example, naturally occurring polymorphic variants have been observed in the retinal specific genes encoding the photoreceptor proteins rhodopsin and peripherin (Table 2). Although these do not occur at appropriate ribozyme cleavage sites (NUX sites in RNA open loop structures) approaches inter alia antisense, triplex helix or antibodies could be utilised to achieve suppression of single alleles carrying disease mutations while enabling continued expression from alternative allelic forms of the gene with wild type sequence using these or other polymorphisms. Additionally further sequencing of these retinal genes in intronic and UTR regions may reveal appropriate polymorphic target sites for ribozymes. The high levels of polymorphism inherent in many human genes are only currently being elucidated as a result of the human genome sequencing project and other major sequencing efforts. Undoubtedly appropriate polymorphic sites will be found enabling specific suppression of one allele of many genes carrying deleterious mutations. This process will be expedited by data provided by projects such as the human genome project - approporiate polymorphisms for suppression effectors targeted either in coding regions or alternatively in non-coding regions which are under less evolutionary constraint than coding regions and therefore show a greater degree of polymorphic variation should become available for most if not all human genes.

In all three examples provided, cDNAs with alternative allelic variants in the regions targeted by ribozymes were generated. RNAs expressed from these cDNAs were protected entirely from cleavage due the absence of the ribozyme target for each of the ribozymes tested. Of particular interest is the fact that a single nucleotide alteration can obliterate a ribozyme target site thereby preventing RNA cleavage. Given the increasing number of such sites being identified together with the continuing elucidation of the molecular pathogenesis of dominant and polygenic diseases the number of targets for this invention is rapidly increasing.

As highlighted before in the text using this invention the same method of suppression (targeting one allele of a gene while allowing continued expression of the other allele) and where necessary gene replacement (using a replacement gene with a different allelic form than that targeted by suppressors to supplement gene expression) may be used as a therapeutic approach for many different mutations within a given gene.

### REFERENCES

Carter G and Lemoine NR. (1993) Cancer Res 67: 869-876.
Cazenave et al. (1989) Nuc Acid Res 17: 4255-4273.
DUAlessio M et al. (1991) Am J Hum Genet 49: 400-406.
Dalgleish R et al. (1986) Hum Genet 73: 91-92.
Dosaka-Akita H et al. (1995) Cancer Res 55: 1559-1564.
Dryja TP et al. (1990) Nature 343: 364-366.
Duval-Valentin et al. (1992) Proc Natl Acad Sci USA 89: 504-508.
Ellis and Rodgers (1993) Nuc Acid Res 21: 5171-5178.
Farrar GJ et al. (1991) Nature 354: 478-480.
Farrar GJ et al. (1991) Genomics 14: 805-807.
Farrar GJ et al. (1995) Invest Ophthamol Vis Sci (ARVO) 36: (4).
Feng M, Cabrera G, Deshane J, Scanlon K and Curiel DT. (1995) Can Res 55: 2024-2028.
Filie et al. (1993) Hum Mut 2: 380-388.
Gaughan DJ, Steel DM, Whitehead SA. (1995) FEBS Letters 374: 241-245.
Hanvey JC et al. (1992) Science 258: 1481-1485.
Hardenbol P and Van Dyke MW. (1996) Proc Natl Acad Sci USA 93: 2811-2816.
Herschlag D, Khosla M, Tsuchihashi Z and Karpel RL. (1994) EMBO 13: (12) 2913-2924.
Herskowitz et al. (1987) Nature 329: 219-222.
Humphries P, Kenna PF nd Farrar GJ. (1992) Science 256: 804-808.
Humphries M et al. (1997) Nat Genet 15: 216-219.
Jankowsky E and Schwenzer B. (1996) Nuc Acid Res 24: (3) 423-429.
Jones JT, Lee S-W and Sullenger BA. (1996) Nature Medicine 2: 643-648.
Jordan SA et al. (1993) Nature Genetics 4: 54-58.
Quattrone A, Fibbi G, Anichini E, Pucci M et al. (1995) Can Res 55: 90-95.
Kajiwara et al. (1991) Nature 354: 480-483.
Knudsen H and Nielsen PE. (1996) Nuc Acid Res 24: (3) 494-500.
Lange W et al. (1993) Leukemia 7: 1786-1794.
Mansergh F et al. (1995) J Med Genet 32: 855-858.
Mashhour B et al. (1994) Gene Therapy 1: 122-126.
McKay RA, Cummins LL, graham MJ, Lesnik EA et al. (1996) Nuc Acid Res 24: (3) 411-417.
McWilliam P et al. (1989) Genomics 5: 612-619.
Ohkawa J, Yuyama N, Takebe Y, Nishikawa S and Taira K. (1993) Proc Natl Acad Sci 90: 11302-11306.
Ohta Y, Kijima H, Ohkawa T, Kashani-Sabet M and Scanlon KJ. (1996) Nuc Acid Res 24: (5) 938-942.
Ott J et al. (1989) Proc Natl acad Sci 87: 701-704.
Oyama T et al. (1995) Pathol Int 45: 45-50.
Phillips CL et al. (1990) J Clin Invest 86: 1723-1728.
Postel et al. (1991) Proc Natl Acad Sci USA 88: 8227-8231.
Porumb H, Gousset , Letellier R, Salle V, et al. (1996) Can Res 56: 515-522.
Rimsky et al. (1989) Nature 341: 453-456.
Sullenger BA and Cech TR. (1994) Nature 371: 619-622.
Sun JS et al. (1989) Proc Natl Acad Sci USA 86: 9198-9202.
Taylor RW et al. (1997) Nat Genetics 15: 212-215.
Trauger JW, Baird EE and Dervan PB. (1996) Nature 382: 559-561.
Valera A et al. (1994) J Biol Chem 269: 28543-28546.
Van Soest S et al. (1994) Genomics 22: 499-504.
Vasan NS et al. (1991) Amer J Hum Genet 48: 305-317.
Wei Z, Tung C-H, Zhu T, Dickerhof WA et al. (1996) Nuc Acid Res 24: (4) 655-661.
Westerhausen AI, Constantinou CD and Prockop DJ. (1990) Nuc Acid Res 18: 4968.
Willing MC et al. (1993) Am J Hum Genet 45: 223-227.
Zhuang J et al. (1996) Hum Mut 7: 89-99.

## Claims

1. An in vitro method for suppressing one allele of an endogenous gene, wherein said allele contains a mutation having a deleterious effect, and introducing a replacement gene, said method comprising the steps of:
(i) providing suppression effector(s) able to suppress expression of an allele of a gene to be suppressed wherein the suppression effector(s) binds a polymorphism in coding region(s) within said gene, wherein said polymorphism is not causally associated with said deleterious effect, and
(ii) providing replacement nucleic acid which is a different naturally occurring allele, wherein the replacement nucleic acid has a sequence which allows it to be expressed and escape complete suppression.

2. The method according to Claim 1, wherein said gene is a rhodopsin gene.

3. The method according to either one of Claims 1 and 2, wherein the suppression effectors are ribozymes.

4. The use of suppression effector(s) able to suppress expression of an allele of a gene to be suppressed, wherein said allele contains a mutation having a deleterious effect, wherein the suppression effector(s) binds a polymorphism in coding region(s) within said gene, wherein said polymorphism is not causally associated with said deleterious effect,
and replacement nucleic acid which is a different naturally occurring allele, wherein the replacement nucleic acid has a sequence which allows it to be expressed and escape complete suppression
in the preparation of a combined medicament for the treatment of an autosomal dominant or polygenic disease.

5. The use as claimed in Claim 4, wherein said gene is a rhodopsin gene.

6. The use as claimed in either one of Claims 4 and 5,
wherein the disease is rhodopsin linked autosomal dominant RP.

7. The use according to any one of Claims 4 to 6 wherein the suppression effectors are ribozymes.

8. The use as claimed in any one of Claims 4 to 7 wherein the suppression effector(s) and / or replacement nucleic acid(s) are provided in at least one vector.

9. The use as claimed in Claim 8 wherein the suppression effector(s) and replacement nucleic acid(s) are provided in the same vector.

10. A kit for use in the treatment of a disease caused by a deleterious mutation in a gene, the kit comprising suppression effectors able to suppress expression of at least one allele to be suppressed, wherein said allele contains a mutation having a deleterious effect, wherein the suppression effectors bind a polymorphism in coding region(s) within said gene to be suppressed, wherein said polymorphism is not causally associated with said deleterious effect, and replacement nucleic acid which is a different naturally occurring allele,
wherein the replacement nucleic acid has a sequence which allows it to be expressed and escape complete suppression.

11. The kit according to Claim 10, wherein said gene is a rhodopsin gene.

12. The kit according to either one of Claims 10 and 11
wherein the suppression effectors are ribozymes.

## Patentansprüche

1. Ein In-vitro-Verfahren zum Supprimieren eines Allels eines endogenen Gens, wobei das Allel eine Mutation mit einem schädlichen Effekt enthält, und Einführen eines Ersatzgens, wobei das Verfahren die folgenden Schritte beinhaltet:
(i) Bereitstellen von einem Suppressionseffektor/von Suppresionseffektoren, der/die dazu in der Lage ist/sind, die Expression eines Allels eines zu supprimierenden Gens zu supprimieren, wobei der
Suppressionseffektor/die Suppressionseffektoren einen Polymorphismus in einer codierenden Region/in codierenden Regionen innerhalb des Gens bindet/binden, wobei der Polymorphismus kausal nicht mit dem schädlichen Effekt in Zusammenhang steht, und
(ii) Bereitstellen von Ersatznukleinsäure, die ein anderes natürlich auftretendes Allel ist, wobei die Ersatznukleinsäure eine Sequenz aufweist, die ihr ermöglicht, exprimiert zu werden und vollständiger Suppression zu entgehen.

2. Verfahren gemäß Anspruch 1, wobei das Gen ein Rhodopsin-Gen ist.

3. Verfahren gemäß einem der Ansprüche 1 und 2, wobei die Suppressionseffektoren Ribozyme sind.

4. Die Verwendung von einem Suppressionseffektor/von Suppresionseffektoren, der/die dazu in der Lage ist/sind, die Expression eines Allels eines zu supprimierenden Gens zu supprimieren, wobei das Allel eine Mutation mit einem schädlichen Effekt enthält, wobei der Suppressionseffektor/die Suppressionseffektoren einen Polymorphismus in der codierenden Region/in den codierenden Regionen innerhalb des Gens bindet/binden, wobei der Polymorphismus kausal nicht mit dem schädlichen Effekt in Zusammenhang steht,
und Ersatznukleinsäure, die ein anderes natürlich auftretendes Allel ist, wobei die Ersatznukleinsäure eine Sequenz aufweist, die ihr ermöglicht, exprimiert zu werden und vollständiger Suppression zu entgehen
bei der Zubereitung eines kombinierten Medikaments für die Behandlung einer autosomal-dominanten oder polygenen Erkrankung.

5. Verwendung gemäß Anspruch 4, wobei das Gen ein Rhodopsin-Gen ist.

6. Verwendung gemäß einem der Ansprüche 4 und 5, wobei die Erkrankung rhodopsingekoppelte autosomal-dominante RP ist.

7. Verwendung gemäß einem der Ansprüche 4 bis 6, wobei die Suppressionseffektoren Ribozyme sind.

8. Verwendung gemäß einem der Ansprüche 4 bis 7, wobei der Suppressionseffektor/die Suppressionseffektoren und/oder die Ersatznukleinsäure/die Ersatznukleinsäuren in mindestens einem Vektor bereitgestellt werden.

9. Verwendung gemäß Anspruch 8, wobei der Suppressionseffektor/die Suppressionseffektoren und die Ersatznukleinsäure/die Ersatznukleinsäuren in dem selben Vektor bereitgestellt werden.

10. Eine Ausstattung zur Verwendung bei der Behandlung einer Erkrankung, die durch eine schädliche Mutation in einem Gen verursacht wird, wobei die Ausstattung Suppressionseffektoren beinhaltet, die in der Lage sind, die Expression von mindestens einem zu supprimierenden Allel zu supprimieren, wobei das Allel eine Mutation mit einem schädlichen Effekt enthält, wobei die Suppressionseffektoren einen Polymorphismus in der codierenden Region/in den codierenden Regionen innerhalb des zu supprimierenden Gens bindet/binden, wobei der Polymorphismus kausal nicht mit dem schädlichen Effekt in Zusammenhang steht, und Ersatznukleinsäure, die ein anderes natürlich auftretendes Allel ist, wobei die Ersatznukleinsäure eine Sequenz aufweist, die ihr ermöglicht, exprimiert zu werden und vollständiger Suppression zu entgehen.

11. Ausstattung gemäß Anspruch 10, wobei das Gen ein Rhodopsin-Gen ist.

12. Ausstattung gemäß einem der Ansprüche 10 und 11, wobei die Suppressionseffektoren Ribozyme sind.

## Revendications

1. Une méthode in vitro de suppression d'un allèle d'un gène endogène, ledit allèle contenant une mutation à effet délétère, et d'introduction d'un gène de remplacement, ladite méthode comprenant les étapes de :
(i) fournir un ou des effecteurs de suppression capables de supprimer l'expression d'un allèle d'un gène devant être supprimé, le ou les effecteurs de suppression se liant à un polymorphisme dans une ou des régions codantes au sein dudit gène, ledit polymorphisme n'étant pas associé de façon causale audit effet délétère, et
(ii) fournir un acide nucléique de remplacement qui est un allèle d'origine naturelle différent, l'acide nucléique de remplacement ayant une séquence lui permettant d'être exprimé et d'échapper à une suppression totale.

2. La méthode selon la revendication 1, dans laquelle ledit gène est un gène de la rhodopsine.

3. La méthode selon l'une ou l'autre des revendications 1 et 2, dans laquelle les effecteurs de suppression sont des ribozymes.

4. L'utilisation d'un ou plusieurs effecteurs de suppression capables de supprimer l'expression d'un allèle d'un gène devant être supprimé, ledit allèle contenant une mutation à effet délétère, le ou les effecteurs de suppression se liant à un polymorphisme dans une ou des régions codantes au sein dudit gène, ledit polymorphisme n'étant pas associé de façon causale audit effet délétère,
et d'un acide nucléique de remplacement qui est un allèle d'origine naturelle différent, l'acide nucléique de remplacement ayant une séquence lui permettant d'être exprimé et d'échapper à une suppression totale,
dans la préparation d'un médicament combiné destiné au traitement d'une maladie autosomique dominante ou polygénique.

5. L'utilisation telle que revendiquée dans la revendication 4, dans laquelle ledit gène est un gène de la rhodopsine.

6. L'utilisation telle que revendiquée dans l'une ou l'autre des revendications 4 et 5, dans laquelle la maladie est la RP autosomique dominante liée à la rhodopsine.

7. L'utilisation selon une quelconque des revendications 4 à 6, dans laquelle les effecteurs de suppression sont des ribozymes.

8. L'utilisation telle que revendiquée dans une quelconque des revendications 4 à 7 dans laquelle le ou les effecteurs de suppression et/ou le ou les acides nucléiques de remplacement sont fournis dans au moins un vecteur.

9. L'utilisation telle que revendiquée dans la revendication 8 dans laquelle le ou les effecteurs de suppression et le ou les acides nucléiques de remplacement sont fournis dans le même vecteur.

10. Un kit destiné à être utilisé dans le traitement d'une maladie causée par une mutation délétère dans un gène, le kit comprenant dès effecteurs de suppression capables de supprimer l'expression d'au moins un allèle devant être supprimé, ledit allèle contenant une mutation à effet délétère, les effecteurs de suppression se liant à un polymorphisme dans une ou des régions codantes au sein dudit gène devant être supprimé, ledit polymorphisme n'étant pas associé de façon causale audit effet délétère, et un acide nucléique de remplacement qui est un allèle d'origine naturelle différent, l'acide nucléique de remplacement ayant une séquence lui permettant d'être exprimé et d'échapper à une suppression totale.

11. Le kit selon la revendication 10, dans lequel ledit gène est un gène de la rhodopsine.

12. Le kit selon l'une ou l'autre des revendications 10 et 11, dans lequel les effecteurs de suppression sont des ribozymes.
